# EUROPÄISCHE PATENTSCHRIFT

(11) **EP 2 464 290 B1**
(45) Veröffentlichungstag und Bekanntmachung des Hinweises auf die Patenterteilung: **31.07.2013**
(21) Anmeldenummer: 10742091.1
(22) Anmeldetag: 03.08.2010
(51) Int. Cl.: A61B 5/151, G01N 33/52, C12Q 1/54, A61B 5/00

(54) **TESTELEMENT ZUR ANALYSE EINER KÖRPERFLÜSSIGKEIT**
TEST ELEMENT FOR ANALYSING A BODY FLUID
ELEMENT TEST POUR L'ANALYSE D'UN LIQUIDE CORPOREL

(30) Priorität: 13.08.2009 EP 09010434
(43) Veröffentlichungstag der Anmeldung: 20.06.2012
(73) Patentinhaber: Roche Diagnostics GmbH, 68305 Mannheim (DE); F.Hoffmann-La Roche AG, 4070 Basel (CH)
(72) Erfinder: HAAR, Hans-Peter, 69168 Wiesloch (DE); HOENES, Joachim, 64673 Zwingenberg (DE)
(74) Vertreter: Durm & Partner
(86) Internationale Anmeldenummer: PCT/EP2010/004748
(87) Internationale Veröffentlichungsnummer: WO 2011/018172

(56) Entgegenhaltungen:
- EP-A- 1 360 931
- DE-A1- 1 598 153
- US-A- 4 452 887

## Beschreibung

Die Erfindung betrifft ein Testelement zur Erzeugung einer Einstichwunde in einem Körperteil, zur Aufnahme einer Körperflüssigkeit aus dem Körperteil und zur Analyse der Körperflüssigkeit auf einen darin enthaltenen Analyten. Auch ein Verfahren zur Herstellung des Testelementes und ein Analysesystem, zu dem ein erfindungsgemäßes Testelement und ein zu dessen Auswertung geeignetes, speziell angepasstes Auswertegerät gehören, sind Gegenstand der Erfindung.

Derartige Testelemente und Analysesysteme werden vor allem für medizinische Analysen verwendet. Sie sind in zahlreichen Varianten zur quantitativen und qualitativen Bestimmung unterschiedlicher Analyten gebräuchlich. Besonders große medizinische und wirtschaftliche Bedeutung haben Systeme zur Bestimmung der Glucosekonzentration im Blut von Diabetikern. Die Erfindung ist insbesondere für solche Systeme geeignet. Sie ist jedoch nicht darauf beschränkt. Ein anderer wichtiger Analyt ist beispielsweise Cholesterin.

Insbesondere richtet sich die Erfindung auf Anwendungsfälle, bei denen die Analyse durch den Patienten selbst durchgeführt wird, um seinen Gesundheitszustand zu überwachen ("home-monitoring"). Dabei kommt es besonders auf eine einfache Handhabung an. Außerdem müssen die Auswertegeräte möglichst klein, leicht und robust sein.

Die für die Analyse notwendige Probe wird üblicherweise durch einen Stich in den Finger oder in ein anderes Körperteil gewonnen. Die Probenflüssigkeit ist Blut und/oder interstitielle Flüssigkeit, wobei nachfolgend ohne Beschränkung der Allgemeinheit auf Blut Bezug genommen wird. Bei den noch heute überwiegend gebräuchlichen Analysesystemen werden für die Probengewinnung und die Analyse getrennte Instrumente verwendet, nämlich ein Lanzettengerät zur Erzeugung einer Wunde in einem Körperteil, aus der Blut austritt, und Analyseelemente, beispielsweise in Form von Teststreifen, die manuell mit dem aus der Wunde ausgetretenen Blut in Kontakt gebracht und anschließend mit Hilfe eines zu dem Analysesystem gehörenden Gerätes ausgewertet werden. Dies erfordert mehrere Handhabungsschritte: Stich in den Finger, Manipulation der Hautoberfläche zur Förderung des Blutaustritts, Kontaktierung des Teststreifens mit dem Bluttropfen und Auswertung mittels des Gerätes. Diese Handhabungsschritte sind nicht nur zeitaufwendig und unangenehm, sondern für viele Patienten, die meist älter und/oder durch Krankheit beeinträchtigt sind, schwer durchzuführen.

Um diese Probleme zu überwinden, wurden Analysesysteme vorgeschlagen, bei denen sämtliche für die Durchführung der Analyse erforderlichen Schritte vom Einstich in den Finger bis zur Anzeige des analytischen Ergebnisses vollautomatisch ablaufen, ohne dass der Benutzer nach dem Einstich weitere Handhabungsschritte durchführen muss. Derartige Systeme werden in der englischsprachigen Literatur auch als "G&M-systems" bezeichnet, weil sie es ermöglichen, die Probe zu gewinnen ("G" für "get") und den analytischen Wert zu messen ("M"). Die meisten G&M-Systeme arbeiten mit Testelementen, die als integrale Bestandteile sowohl ein Stechelement als auch ein für die Durchführung der Analyse erforderliches Element aufweisen. Die beiden Bestandteile der Testelemente (Stechelement und Analyseelement) werden in der Regel schon beim Hersteller zu dem Testelement assembliert. Es gibt aber auch G&M-Systeme, bei denen das Analyseelement und das Stechelement erst in dem Gerät derartig zusammengebracht werden, dass zwischen beiden Elementen ein Kontakt hergestellt wird, der den Übertritt von Probenflüssigkeit von dem Stechelement zu dem Analyseelement ermöglicht und als "Fluidkontakt" bezeichnet wird.

G&M-Systeme sind beispielsweise in den folgenden Publikationen beschrieben:
(1) WO 01/72220
(2) WO 03/009759
(3) EP 1 360 931 A1
(4) EP 1 360 933 A1
(5) WO 2005/084546
(6) WO 2006/105968
(7) WO 2007/045412

Die Funktion der Testsysteme wird entscheidend von den Eigenschaften der darin verwendeten Testelemente beeinflusst. Sie sollen eine Reihe schwieriger und teilweise widersprüchlicher Anforderungen erfüllen. Einerseits soll eine hohe Analysegenauigkeit erreicht werden. Andererseits soll die Konstruktion möglichst einfach sein und eine kostengünstige Fertigung ermöglichen. Um möglichst viele Testelemente in einem Gerät unterbringen zu können, sollen sie möglichst klein sein. Voraussetzung für eine schnelle und zuverlässige Analyse ist, dass nur ein sehr kleines Probenvolumen von typischerweise weniger als 1 µl benötigt wird. Schließlich müssen die Testelemente und die zugehörigen Auswertegeräte robust genug sein, um im Alltagsbetrieb eine zuverlässige Funktion zu gewährleisten.

Der Erfindung liegt auf dieser Grundlage das technische Problem zugrunde, ein Testelement und zugehöriges Testsystem zur Verfügung zu stellen, das hinsichtlich der vorstehend erörterten Anforderungen verbesserte Eigenschaften aufweist.

Dieses technische Problem wird gelöst durch ein Testelement zum Erzeugen einer Einstichwunde in einem Körperteil, zur Aufnahme einer Körperflüssigkeitsprobe aus dem Körperteil und zur Analyse mittels eines Reagenzsystems, dessen Reaktion mit einem in der Körperflüssigkeit enthaltenen Analyten zu einer Änderung einer an dem Testelement optisch messbaren für das gewünschte analytische Ergebnis charakteristischen Messgröße führt, umfassend ein Stechelement und ein Testfeld, das mindestens einen Teil des Reagenzsystems enthält, wobei das Stechelement eine an einem Ende des Stechelementes angeordnete Spitze zum Erzeugen einer Wunde in dem Körperteil und eine Kapillarstruktur aufweist, die mit dem Bereich der Spitze derart in Fluidverbindung steht, dass nach dem Einstechen der Spitze des Stechelementes in die Haut Körperflüssigkeit in die Kapillarstruktur eindringt, und das Stechelement und das Testfeld relativ zueinander so in einer Flüssigkeitstransferposition positionierbar sind, dass das Testfeld in Fluidkontakt zu einem Teil der Kapillarstruktur des Stechelementes steht, wobei in die Kapillarstruktur eingedrungene Körperflüssigkeit zu dem Testfeld transferiert werden kann. Das Testfeld umfasst eine transparente Tragschicht und eine auf der Tragschicht durch Beschichten angebrachte Testschicht, wobei die von der Tragschicht abgewandte Seite der Testschicht deren Flüssigkeitseintrittsseite bildet, die in der Flüssigkeitstransferposition der Kapillarstruktur zugewandt ist. In der Flüssigkeitstransferposition grenzt die Flüssigkeitseintrittsseite der Testschicht somit unmittelbar und direkt an die Kapillarstruktur an. Der Flüssigkeitsübertritt aus der durch die Kapillarstruktur gebildeten Flüssigkeitshaltestruktur in die Testschicht erfolgt direkt, ohne Vermittlung durch etwas Drittes, insbesondere ohne ein weiteres zwischen der Kapillarstruktur und der Testschicht lokalisiertes strukturelles Element.

Das Stechelement hat bevorzugt eine längliche Form, wobei ein Ende des Stechelementes mit der zum Einstechen in ein Körperteil geeigneten Spitze geformt ist. Die Spitze befindet sich vorzugsweise an einem Punkt, in den die Seitenflächen des länglichen Stechelementes zusammenlaufen, wobei die an der Spitze endenden Seitenflächen zusätzlich geschärfte Kanten aufweisen können. Bei der Benutzung dringt das in Stichrichtung vordere Ende des Stechelementes, beginnend mit der Spitze, in die Haut ein. Danach dringt die Körperflüssigkeit in die Kapillarstruktur und wird in dieser weiter transportiert bis zu dem Teil des Stechelementes, an dem die Übertragung auf das Testfeld stattfindet und der auch als Flüssigkeitstransferbereich bezeichnet wird. Das Eindringen der Flüssigkeit muss nicht nur unmittelbar an der Spitze erfolgen. Es sind sogar Ausführungsformen möglich, bei denen die Spitze selbst geschlossen ist und die Kapillarstruktur hinter der Spitze eine Öffnung für das Eindringen der Körperflüssigkeit aufweist. Der Teil des Stechelementes, der in die Haut eindringt und an dem die Körperflüssigkeit in die Kapillarstruktur eintritt, wird nachfolgend auch als Spitzenbereich oder Bereich der Spitze des Stechelementes bezeichnet. Die Kapillarstruktur erstreckt sich in dem Stechelement von dem Spitzenbereich zu dem Flüssigkeitstransferbereich und ermöglicht somit, dass nach dem Einstechen des Stechelementes in das Körperteil Körperflüssigkeit in die Kapillarstruktur eindringt und vom Bereich der Spitze zu dem Flüssigkeitstransferbereich transportiert wird. Der Flüssigkeitstransferbereich der Kapillarstruktur kann mit dem Bereich der Spitze teilweise oder vollständig überlappen oder befindet sich in einem zum Bereich der Spitze distal gelegenen Bereich des Stechelements.

Die Kapillarstruktur ist im einfachsten Fall - wie bei den meisten der aus dem Stand der Technik bekannten Stechelemente - ein einfacher Kanal. Gemäß einer bevorzugten Ausgestaltung, die später noch näher erläutert wird, schließt die Kapillarstruktur eine zweidimensionale Matrixstruktur mit einer Mehrzahl von Zellen ein. Allgemein ist als Kapillarstruktur jede Gestaltung des Stechelementes (als Teil des Testelementes) geeignet, durch die die Probenflüssigkeit, von Kapillarkräften getrieben, in dem Stechelement zu dem Flüssigkeitstransferbereich transportiert wird. Die Kapillarstruktur kann über mindestens einen Teil iher Länge rillenförmig bzw. halboffen oder auch als zumindest teilweise geschlossener Kanal ausgebildet sein. Der Flüssigkeitstransferbereich kann am Ende der Kapillarstruktur (z.B. am Ende der Rille oder des zumindest teilweise geschlossenen Kanals) oder in einem zugänglichen, also für den Flüssigkeitstransfer geeigneten, Bereich der Kapillarstruktur zwischen dem Anfang der Kapillarstruktur (im Bereich der Spitze des Stechelements) und dem Ende der Kapillarstruktur (z.B. in der Umgebung einer halboffenen Rillenstruktur oder eines offenen Bereichs des teilweise geschlossenen Kanals) sein. Um die erforderliche Kapillarwirkung zu gewährleisten, besteht die Kapillarstruktur üblicherweise aus einem hydrophilen oder hydrophilisierten Material.

Das Testelement muss nicht bereits bei der Herstellung so assembliert worden sein, dass sich seine Bestandteile Stechelement und Testfeld in der Flüssigkeitstransferposition befinden. Der Flüssigkeitstransfer von dem Stechelement zu der Testschicht des Testfeldes muss also nicht ohne weiteres, insbesondere nicht ohne Relativbewegung dieser Elemente zueinander, stattfinden, sobald eine hinreichende, Menge Körperflüssigkeit in die Kapillarstruktur des Stechelementes eingedrungen ist. Vielmehr umfasst die Erfindung auch Ausführungsformen, bei denen das Testfeld und die Kapillarstruktur des Stechelementes nach der Herstellung des Testelementes räumlich getrennt sind und erst in dem Analysegerät in die Flüssigkeitstransferposition gebracht werden, in der sie in Fluidkontakt zueinander stehen. Dabei können das Stechelement und das Testfeld auch zu separaten Teilen des Testelementes gehören, die (beispielsweise in Form eines Stechelementmagazins und eines Analysemagazins) in das Analysegerät eingesetzt werden. Der Begriff "Testelement" ist deshalb allgemein so zu verstehen, dass er jede Konfektionierung umfasst, bei der ein Stechelement und ein Testfeld in eine Flüssigkeitstransferposition bringbar sind, in der sie in Fluidkontakt zueinander stehen. Auch aus zwei Teilelementen bestehende Ausgestaltungen sind möglich, soweit die Teilelemente zur gemeinsamen Verwendung in einem Analysegerät ausgebildet sind. Ebenfalls möglich sind Ausgestaltungen, bei denen das Testfeld an dem Stechelement derartig fixiert ist, dass der Fluidkontakt permanent besteht, also ohne eine Relativbewegung beider Bestandteile des Testelementes ein Flüssigkeitsübertritt stattfindet, sobald eine hinreichende Menge Körperflüssigkeit in die Kapillarstruktur des Stechelementes eingedrungen ist.

Die Analyse erfolgt mittels eines Reagenzsystems, das in der Regel aus mehreren Reagenzien und Hilfsstoffen besteht, die in das Testelement integriert sind und deren Reaktion mit einem in der Körperflüssigkeit enthaltenen Analyten zu einer Änderung einer an dem Testelement messbaren, für das gewünschte analytische Ergebnis charakteristischen Messgröße führt.

Nähere Einzelheiten können der einschlägigen Literatur entnommen werden. Eine Übersicht gibt beispielsweise der Artikel von J. Hönes et al "The Technology Behind Glucose Meters: Test Strips", in Diabetes Technology & Therapeutics, 2008, Supplement 1, pp S 10 bis S 26.

Die Erfindung richtet sich speziell auf sogenannte optische Analyseelemente und -systeme, bei denen die für die Analyse charakteristische Änderung des Testelementes optisch messbar ist. In der Regel führt bei optischen Testelementen die Reaktion zur Änderung der Farbe einer Schicht oder Fläche, die Bestandteil des Testelementes ist und als Detektionsschicht oder Detektionsfläche bezeichnet wird. Die Farbänderung der Detektionsschicht wird photometrisch vermessen. Neben diesen "colorimetrischen" Testsystemen sind auch andere optisch auswertbare Testsysteme bekannt, beispielsweise Systeme, bei denen die Messgröße ein Fluoreszenzsignal ist. Die Erfindung bezieht sich allgemein auf Analysesysteme, bei denen die Analyse auf der Messung einer an dem Testelement optisch messbaren Messgröße beruht, wobei das Ergebnis der optischen Messung für das gewünschte analytische Resultat charakteristisch ist. Nachfolgend wird ohne Beschränkung der Allgemeinheit auf Farbänderungen als Beispiel für optisch messbare Messgrößen Bezug genommen.

Bekannte optische Analyseelemente haben eine Tragstruktur, die in der Regel aus Kunststoff besteht und meist als schmaler, länglicher Kunststoffstreifen ausgebildet ist ("Teststreifen"). Ein Teilbereich des Testelementes, in welchem mindestens ein Teil der Reagenzien lokalisiert ist, wird als Testfeld (englisch: "chemistry pad") bezeichnet. Das Testfeld kann aus einer oder mehreren Schichten bestehen, die in Fluidkontakt zueinander stehen und in der Regel parallel zueinander verlaufen.

Reagenzienhaltige Schichten des Testfeldes bestehen bei gebräuchlichen Analyseelementen oft aus einem saugfähigen porösen Schichtmaterial (beispielsweise Papier, Vlies oder eine Kunststoffmembran), wobei die Reagenzien in die Poren der Schicht integriert sind. Die Reagenzien werden bei der Herstellung der Testschicht in die Poren des Schichtmaterials eingebracht und liegen dort in löslicher oder (beispielsweise durch kovalente Bindung) an die Festphase gebundener Form vor. Jedenfalls bildet die poröse Schicht dabei eine feste Trägermatrix für die Reagenzien, die auch erhalten bleibt, wenn die Probenflüssigkeit in die Matrix eindringt und die Reagenzien löst. Deswegen kann man diesen Testschicht-Typ auch als Matrix-Testschicht bezeichnen. Die wässrige Probenflüssigkeit wird bei diesem Testschicht-Typ also vom porösen Schichtmaterial aufgenommen und so erst in Kontakt mit den Reagenzien gebracht.

Bei der Erfindung wird ein anderer bekannter Testfeldtyp verwendet, bei dem eine Testschicht auf ein geeignetes Trägermaterial durch Beschichten aufgebracht ist ("coated test layer"). Nachfolgend wird eine solche Testschicht auch als CTL-Schicht bezeichnet. Zur Herstellung einer CTL-Schicht, die auch als "Testfilm" bezeichnet wird, werden die Reagenzien mit einem Binde- oder Verdickungsmittel gemischt, um eine zähflüssige Beschichtungsmasse zu bilden. Nach dem Beschichten und Trocknen wird ein dünner Film auf dem transparenten Trägermaterial gebildet. Das Verdickungs- oder Bindemittel wird deshalb auch als "Filmbildner" bezeichnet. Die CTL-Schicht wird auf den transparenten und nicht porösen Träger aufgebracht (beschichtet), ohne dass Filmbildner oder Reagenzien in das Trägermaterial eindringen. Die CTL-Schicht muss so ausgebildet sein, dass bei Kontakt mit der wässrigen Probenflüssigkeit die für die Analyse erforderliche Reaktion des in der Probenflüssigkeit enthaltenen Analyten mit den in der CTL-Schicht enthaltenen Reagenzien stattfindet. Um dies zu gewährleisten ist eine bevorzugte CTL-Schicht zumindest teilweise löslich und/oder quellfähig. Ein charakteristisches Merkmal einer solchen CTL-Schicht besteht darin, dass sie, im Gegensatz zu den erwähnten Matrix-Testschichten, keine auch nach dem Eindringen der wässrigen Probenflüssigkeit dauerhaft feste poröse Struktur hat. Um für derartige Testfelder dennoch eine Flüssigkeitshaltestruktur zu bieten, werden im Stand der Technik Netzstrukturen oder Vliese verwendet, in die die Probenflüssigkeit eintreten kann und die als zusätzliches, an die CTL-Schicht angrenzendes Element des Testfeldes eine Flüssigkeitssäule mit einer definierten Höhe und einem definierten Volumen bereit stellen. Im Gegensatz zum Stand der Technik weisen erfindungsgemäße Testfelder keine zusätzliche Flüssigkeitshaltestruktur (eines flüssigkeitsbeständigen Schichtmaterials) auf. Die Flüssigkeitssäule der Probenflüssigkeit mit einer definierten Höhe und einem definierten Volumen, die in der Flüssigkeitstransferposition mit der CTL-Schicht direkt und unmittelbar in Kontakt steht, wird erfindungsgemäß durch die Kapillarstruktur des Stechelementes zur Verfügung gestellt.

Für die Erfindung sind insbesondere enzymatische Reagenzsysteme geeignet. Sie enthalten ein Enzym, das spezifisch mit dem Analyten reagiert. Im Falle der Glucoseanalyse wird als Enzym beispielsweise Glucosedehydrogenase (GDH) verwendet, wobei im Rahmen der Erfindung besonders bevorzugt PQQ-abhängige GDH eingesetzt wird, welche auch als GlucDOR (glucose dye oxidoreductase) bezeichnet wird. Die Reaktion zwischen Glucose und Enzym führt zu einer weiteren Reaktion eines Farbbildungsreagenz (Indikator), die mit einer Farbänderung verbunden ist. Bevorzugt enthält das Reagenzsystem weitere Reaktionsbestandteile, insbesondere einen Mediator, der den mit der Reaktion verbundenen Elektronentransfer von dem Enzym auf den Indikator erleichtert und dadurch eine schnellere Analyse ermöglicht. Auch dies wird in der einschlägigen Literatur, beispielsweise in der zitierten Publikation von J. Hönes et al., näher erläutert.

Bei dem erfindungsgemäßen Testelement umfasst das Testfeld eine transparente Tragschicht und eine auf dieser Tragschicht durch Beschichten angebrachte Testschicht (CTL-Schicht) ohne eine zusätzliche Flüssigkeitshaltestruktur. Erfindungsgemäß wird die Flüssigkeitshaltestruktur durch das Stechelement in der Flüssigkeitstransferposition bereitgestellt. In dem Testelement ist das Testfeld so orientiert, dass die von der Tragschicht abgewandte Seite der CTL-Schicht die Flüssigkeitseintrittsseite bildet und diese in der Flüssigkeitstransferposition der Kapillarstruktur des Stechelementes zugewandt ist, so dass in der Flüssigkeitstransferposition ein direkter und unmittelbarer Kontakt zwischen der in der Kapillarstruktur des Stechelementes bereit gestellten Körperflüssigkeit und der CTL-Schicht erfolgen kann. Die CTL-Schicht kann aus mehreren Teilschichten bestehen. Vorzugsweise schließt sie zwei Teilschichten ein, wobei eine erste auf die Tragschicht beschichtete Teilschicht eine Reaktionsschicht ist, die mindestens einen Teil der Reagenzien des Reagenzsystems enthält, und eine zweite auf die Reaktionsschicht beschichtete Teilschicht eine opake Lichtblockierungsschicht ist. Sie enthält ein Pigment (beispielsweise TiO₂), durch das der Durchtritt von Licht durch die Lichtblockierungsschicht weitgehend verhindert wird. Vorzugsweise ist die Korngröße des Pigments so bemessen, dass Erythrozyten nicht oder nur in geringem Umfang durch die Lichtblockierungsschicht hindurch treten können.

Bei der Analyse wird die Probenflüssigkeit durch die Kapillarstruktur zu dem Flüssigkeitstransferbereich transportiert. Wenn sich das Stechelement und das Testfeld in der Flüssigkeitstransferposition befinden, dringt die Probenflüssigkeit (senkrecht zu der Testfeldebene) in die benachbarte Testschicht ein und löst die darin enthaltenen Reagenzien, so dass die vorgesehenen Reaktionen des Reagenzsystems mit dem Analyten stattfinden. Dies führt zu einer Farbänderung in der Testschicht. Aufgrund von Konzentrationsgradienten zwischen Testschicht und Probenflüssigkeit kommt es zu Diffusionsprozessen reaktiver Bestandteile der Testschicht sowie abreagierter Bestandteile der Testschicht (Produkte) in die wässrige Probenflüssigkeit. Die optische Messung der Farbänderung erfolgt (vorzugsweise reflexionsfotometrisch) durch die transparente Tragschicht hindurch, bevorzugt zu einem Zeitpunkt, in dem sich ein quasi-stationärer Zustand bei besagten Diffusionsvorgängen eingestellt hat. Die Detektionsschicht wird demzufolge von der CTL-Schicht, vorzugsweise von deren Reaktionsschicht, gebildet.

Bei der bevorzugten Ausführungsform mit Lichtblockierungsschicht wird eine Störung der optischen Messung durch den in der Probe enthaltenen roten Blutfarbstoff Hämoglobin weitgehend ausgeschlossen, weil die Lichtblockierungsschicht das Messlicht blockiert und (vorzugsweise) gleichzeitig auch den Durchtritt von Erythrozyten aus der in der Kapillarstruktur befindlichen Flüssigkeitsschicht in die Reaktionsschicht mindestens weitgehend verhindert. Diese Verfahrensweise zur Unterdrückung der Hämoglobin-Störung bei optischen Tests und nähere Einzelheiten über geeignete Lichtblockierungsschichten sind aus der einschlägigen Literatur, beispielsweise dem US-Patent 5,846,837, bekannt.

Bei den optischen Tests, auf die sich die Erfindung bezieht, findet die für die Analyse charakteristische Farbbildungsreaktion lokalisiert in der CTL-Schicht statt. Im Falle des bevorzugten zweischichtigen Aufbaus der CTL-Schicht konzentriert sich die Farbbildung in der - im Vergleich zur Lichtblockierungsschicht transparenteren - Reaktionsschicht, die deswegen auch als Farbbildungsschicht bezeichnet werden kann. Dieser Effekt resultiert zunächst daraus, dass sich die CTL-Schicht bei Kontakt mit der wässrigen Probenflüssigkeit nur teilweise und langsam löst. Zum Zeitpunkt der Messung hat sie eine zähflüssige Konsistenz und ist an der Tragschicht lokalisiert. Die Farbbildung findet demzufolge in der Detektionsschicht und in unmittelbarer Nähe zur transparenten Tragschicht statt.

Die damit erzielten vorteilhaften Wirkungen werden im Vergleich zu dem Dokument (3), EP 1360931 A1, deutlich. Darin wird ein Testelement beschrieben, das im Wesentlichen aus einem Stechelement mit einer als "Matrixbereich" (matrix area) bezeichneten muldenförmigen Vertiefung und einer die Vertiefung überspannenden Deckschicht ("top layer") besteht.

Bei einer ersten Ausführungsform ist die Deckschicht transparent, also nicht porös. Die Reagenzien des Farbbildungssystems ("signal producing system") werden in den Matrixbereich der muldenförmigen Vertiefung eingebracht.

Wenn die Probenflüssigkeit bei der Durchführung eines Tests in die muldenförmige Vertiefung eindringt, findet die Farbbildung in der muldenförmigen Vertiefung statt und kann durch die transparente Deckschicht beobachtet werden. Infolge des großen Volumens der muldenförmigen Vertiefung ist für eine ausreichende Farbbildung eine große Reagenzmenge und eine lange Reaktionszeit erforderlich.

Bei einer zweiten Ausführungsform des Dokumentes (3) ist vorgesehen, dass die Deckschicht porös, somit nicht transparent, ist. Dabei wird der für die chemische Reaktion des Farbbildungssystems erforderliche Raum durch die Poren der Deckschicht zur Verfügung gestellt. Die Deckschicht besteht aus einem saugfähigen porösen Schichtmaterial, in dessen Poren die Reagenzien imprägniert werden. Sie ist eine Matrix-Testschicht in dem oben erläuterten Sinn. Die Farbbildung kann an der Oberfläche der Testschicht nach dem Eindringen der Probenflüssigkeit und Ablaufen der farbbildenden Reaktion beobachtet werden. Dieser Vorgang ist langsam und erfordert eine entsprechend lange Testzeit. Im Vergleich zu dem erfindungsgemäßen Test muss eine höhere Reagenzmenge eingesetzt werden, um eine ähnlich intensive Farbbildung und damit ähnliche Genauigkeit des Tests zu erreichen.

Erfindungsgemäß findet die Reaktion und Farbbildung räumlich hochkonzentriert hauptsächlich in der CTL-Schicht statt, Diesbezüglich ist es besonders vorteilhaft, wenn gemäß einer bevorzugten Ausführungsform der Erfindung mindestens ein Bestandteil des Reagenzsystems, insbesondere das Enzym oder der Mediator, besonders bevorzugt das Enzym, an eine andere Komponente der Testschicht, insbesondere einen in der Testschicht enthaltenden Füllstoff (also eine an der Reaktion nicht beteiligte Komponente der Testschicht) gebunden ist. Diese Bedingung ist insbesondere erfüllt, wenn man GlucDOR zusammen mit einem negativ geladenen Bestandteil der CTL-Schicht (z.B. auf Basis von Natrium-Aluminium-Silikat) verwendet. Das Enzym ist stark positiv geladen, so dass sich eine auf elektrostatischen Kräften basierende Bindung ergibt. Es können jedoch auch andere Bindungsmechanismen, beispielsweise auf der Grundlage einer kovalenten Bindung mit anderen (insbesondere an der Reaktion nicht beteiligten) Komponenten der CTL-Schicht, verwendet werden. Durch diese Bindung wird die Diffusion des betreffenden Bestandteils des Reagenzsystems nach dem Eindringen der Probenflüssigkeit verlangsamt und damit die Lokalisierung in der Nähe der transparenten Tragschicht verbessert.

Diese Lokalisierung der Reaktion und Farbbildung ist ein Grund, warum die bei der Erfindung verwendeten optischen Testverfahren es ermöglichen, mit extrem kleinen Probenmengen hochempfindliche Tests zu realisieren. Die Testschicht ist vorzugsweise extrem dünn (bevorzugt höchstens 20 µm) und benötigt deshalb nur eine sehr kleine Probenmenge, bezogen auf die Fläche der Testschicht (vorzugsweise höchstens 0,1 µl/mm², besonders bevorzugt höchstens 0,05 µl/m²). Durch den Kontakt der wässrigen Probenflüssigkeit mit der Testschicht kommt es zum Aufquellen und/oder Lösen von Bestandteilen der Testschicht und somit zu Diffusionsprozessen reaktiver Bestandteile der Testschicht sowie abreagierter Bestandteile der Testschicht (Produkte) in die wässrige Probenflüssigkeit und ebenfalls - wenn auch zunächst in geringerem Maße - in umgekehrter Richtung von der Probenflüssigkeit in die Testschicht. Trotzdem ist die Konzentration der Reaktionsteilnehmer im Bereich der Detektionsfläche hoch und die Farbbildung intensiv. Insofern unterscheidet sich der erfindungsgemäße optische Test grundlegend von elektrochemischen Tests, bei denen die Reagenzien innerhalb der mit Elektroden ausgestatteten Reaktionskammer (Elektrodenkammer) gelöst werden, so dass die Größe des Reaktionsvolumens von der Größe der Elektrodenkammer bestimmt wird.

Je größer das über dem Testfeld zur Verfügung stehende Probenvolumen ist, desto länger andauernd sind die Diffusionsprozesse zwischen den reaktiven Bestandteilen der Testschicht und der Probenflüssigkeit. Eine reproduzierbare Messung einer Konzentration eines Analyten kann folglich erst dann erfolgen, wenn sich sowohl die Reaktion als auch die Diffusionsprozesse in einem quasi-stationären Zustand befinden, so dass sich ein zur Auswertung eines erfindungsgemäßen Testelementes vorgesehenes Messgerät entsprechend kalibrieren lässt. Dies setzt voraus, dass der quasi-stationäre Zustand der Reaktion und der Diffusionsprozesse bei jeder Messung gleich ist. Dies wird zum einen dadurch gewährleistet, dass die Testchemie der CTL-Schicht entsprechend reproduzierbar auf die Tragschicht aufgebracht wird. Da die Einstellung eines quasi-stationären Zustandes der Diffusionsprozesse vom Volumen abhängt, muss zum anderen die Höhe der Flüssigkeitssäule und somit das Volumen der Probenflüssigkeit oberhalb des Testfeldes so dimensioniert sein, dass das Volumen für den Diffusionsprozess als unendlich anzusehen ist. Dann kann die Vermessung des Systems volumenunabhängig erfolgen.

Im Rahmen der Erfindung wurde festgestellt, dass es trotz des Bestrebens nach einem möglichst kleinen Probenvolumen vorteilhaft ist, das Testelement so zu konstruieren, dass die Flüssigkeitseintrittsseite des Testfeldes während der Reaktion mit einer Flüssigkeitsschicht in Kontakt steht, deren Schichtdicke einen Mindestwert hat, um den Einfluss von Diffusionsprozessen auf den Verlauf und die optische Messung der Reaktion zwischen den in der Probenflüssigkeit vorhandenen Reaktanden und der Testchemie des Testfeldes zu minimieren, so dass die Messung volumenunabhängig erfolgen kann. Dabei kommt es auf die Dimension der Flüssigkeitsschicht senkrecht zu der Testfeldebene an. Diese Abmessung wird nachfolgend auch als Flüssigkeitssäule bezeichnet.

Die Höhe der Flüssigkeitssäule (d.h. die Dicke der Flüssigkeitsschicht) determiniert hierbei die Dauer der Messzeit, für die das Testelement ausgebildet ist. Die Höhe der Flüssigkeitssäule sollte also derart bemessen sein, dass die oben beschriebenen Diffusionsprozesse zwischen der wässrigen Probenflüssigkeit und den Bestandteilen der Reaktionsschicht des Testelements nur einen vernachlässigbaren Einfluss auf den Verlauf und das Ergebnis der optischen Messung haben. Trotz des Bestrebens nach möglichst kleinen Probenvolumen und einer verkürzten Messzeit, d.h. der Zeit zwischen der ersten Kontaktierung des Testfeldes mit der Probenflüssigkeit und der optischen Messung (im Falle mehrerer optischer Messungen der letzten optischen Messung), muss also eine notwendige Mindesthöhe der Flüssigkeitssäule gewährleistet sein, um die gewünschte Unabhängigkeit von Diffusionsvorgängen zu erreichen. Die erforderliche Mindesthöhe der Flüssigkeitssäule und somit auch das erforderliche Volumen oberhalb des Testfeldes hängt von der jeweiligen Testchemie sowie der Struktur und Dicke der CTL-Schicht ab. Beispielsweise können zu dieser Korrelation folgende Zahlenangaben gemacht werden:
- Die Kapillarstruktur sollte so ausgebildet sein, dass die Schichtdicke der an die Flüssigkeitseintrittsseite des Testfeldes angrenzenden durch die Kapillarstruktur bestimmten Flüssigkeitsschicht mindestens 100 µm beträgt, wenn das Testelement für eine Messzeit von maximal 15 Sekunden ausgebildet ist.
- Die Kapillarstruktur sollte so ausgebildet sein, dass die Schichtdicke der an die Flüssigkeitseintrittsseite des Testfeldes angrenzenden durch die Kapillarstruktur bestimmten Flüssigkeitsschicht mindestens 50 µm beträgt, wenn das Testelement für eine Messzeit von maximal 5 Sekunden ausgebildet ist.
- Die Kapillarstruktur sollte so ausgebildet sein, dass die Schichtdicke der an die Flüssigkeitseintrittsseite des Testfeldes angrenzenden durch die Kapillarstruktur bestimmten Flüssigkeitsschicht mindestens 20 µm beträgt, wenn das Testelement für eine Messzeit von maximal 1 Sekunde ausgebildet ist.

Wesentlich ist also, dass in Abstimmung mit der Testchemie und Struktur der CTL-Schicht die Höhe der Flüssigkeitssäule so groß gewählt wird, dass im Wesentlichen keine durch Diffusionsprozesse bedingte Veränderung der Messergebnisse auftreten, d.h. die Messung unabhängig vom Volumen erfolgen kann.

Diese Minimalbedingung hinsichtlich der Flüssigkeitssäule erfordert ein zusätzliches Volumen. Dennoch können erfindungsgemäße Testelemente extrem klein konstruiert werden, weil die erforderliche Flüssigkeitssäule nicht in dem Analyseteil des Testelementes, sondern in dem Stechelement zur Verfügung gestellt wird. Dabei bildet die in das Stechelement integrierte Kapillarstruktur eine "Flüssigkeitshaltestruktur", in der die erforderliche Flüssigkeitssäule in Kontakt mit dem optischen Testfeld vorrätig gehalten wird. Die Flüssigkeit steht in direktem Diffusionsaustausch mit der CTL-Schicht und den darin enthaltenen Reagenzien des Reagenzsystems. Hierbei muss sich nicht zwangsläufig ein vollständiges Diffusionsgleichgewicht einstellen. Es ist hinreichend, wenn der Diffusionsaustausch derart erfolgt, dass eine reproduzierbare optische Messung ermöglicht wird.

Die in das Stechelement integrierte Kapillarstruktur hat somit zwei Funktionen. Zum einen sorgt sie für den Transport der Körperflüssigkeit vom Bereich der Spitze des Stechelements zum distalen Ende des Stechelementes. Zum anderen fungiert sie als ,Flüssigkeitshaltestruktur' und stellt sicher, dass Körperflüssigkeit aus der Kapillarstruktur in das Testelement übertreten kann und dass mindestens bis zum Abschluss der Messung ein vordefinierter Mindestwert der Flüssigkeitsschichtdicke in dem Bereich der Benetzung erhalten bleibt und während der Messung an dem Testfeld zur Verfügung steht. Somit gelingt es, dass trotz des Auftretens von Diffusionsvorgängen zwischen Bestandteilen der Reaktionsschicht des Testfeldes und der in der Kapillarstruktur vorgehaltenen Körperflüssigkeit die erwünschte Messgenauigkeit erzielt wird. Eine zusätzliche Flüssigkeitshaltestruktur wie bei Testelementen des Standes der Technik ist nicht erforderlich. Stattdessen wird diese Funktion durch das Stechelement, das bevorzugt aus Metall hergestellt ist, gebildet.

In der Flüssigkeitstransferposition grenzt die Testschicht mit ihrer Flüssigkeitseintrittsseite unmittelbar an die Kapillarstruktur an. Zwischen der Flüssigkeitseintrittsseite der Testschicht und der Kapillarstruktur befindet sich also keine weitere Schicht, insbesondere keine Flüssigkeitshaltestruktur, wie beispielsweise ein Netz oder Vlies. Die einzige Flüssigkeitshaltestruktur des Testelementes wird von der Kapillarstruktur in dem Testelement gebildet, d.h. die Flüssigkeit in der Kapillarstruktur des Testelements steht dann in direktem Diffusionsaustausch mit der CTL-Schicht des Testfeldes. Die CTL-Schicht enthält keine porösen Bestandteile, die als Flüssigkeitshaltestruktur dienen könnten.

Das Testfeld kann sowohl in seiner Flächenausdehnung, als auch in seiner Dicke extrem kleine Abmessungen haben. Es ist bevorzugt so in das Testelement integriert, dass sein tragendes Element das Stechelement ist. Insofern unterscheidet es sich grundlegend von einem vollständigen Teststreifen (oder sonstigen Analyseelementen), wie er bei früheren Konstruktionen eines G&M-Testelementes verwendet wurde.

Insgesamt werden durch die Erfindung entscheidende Vorteile erreicht:
- Die Konstruktion ist einfach und kostengünstig herzustellen, wie noch näher erläutert wird.
- Trotz der Mindestgröße der Flüssigkeitshalteschicht ergibt sich insgesamt eine extrem kleine Bauweise. Dies führt nicht nur zu einem geringen erforderlichen Probenvolumen, sondern trägt auch zu einer hohen Empfindlichkeit des Tests bei.

Das erfindungsgemäße Testelement ist so ausgebildet, dass wenigstens zu einem Übertragungszeitpunkt die Kapillarstruktur des Stechelements derart an die Testschicht des Testfelds angenähert ist, dass ein Flüssigkeitsübertrag von der Kapillarstruktur (direkt) auf das Testfeld stattfindet. Zu dem Übertragungszeitpunkt befindet sich das Testfeld also in der Flüssigkeitstransferposition, in der die Flüssigkeitseintrittsseite der Testschicht unmittelbar an die Kapillarstruktur angrenzt. Insbesondere ist zwischen der Kapillarstruktur des Testelementes und der Testschicht keine zusätzliche Flüssigkeitshaltestruktur vorhanden. Vor oder nach dem Übertragungszeitpunkt können sich Stechelement und Testfeld relativ zueinander in einer Position befinden, in der kein Flüssigkeitstransport möglich ist. Sie müssen aber in einer solchen Flüssigkeitstransferposition positionierbar sein.

Die in dem Testfeld fehlende Flüssigkeitshaltestruktur wird erfindungsgemäß durch die Kapillarstruktur des Stechelements zur Verfügung gestellt. Da die Flüssigkeitshaltestruktur erst beim Übertrag der Flüssigkeit auf das Testfeld vorhanden sein muss, genügt eine Annäherung oder Kontaktierung von Stechelement und Testfeld erst im oder kurz vor dem Übertragungszeitpunkt der Flüssigkeit. Bei dieser Ausführungsform wird das zur Analyse notwendige Element erst dann vervollständigt, wenn das Stechelement derart angenähert ist, dass die Kapillarstruktur eine Flüssigkeitshaltestruktur für das Testfeld zur Verfügung stellt und Blut von dem Stechelement auf das Testfeld gelangt.

In einer besonders bevorzugten Ausführungsform umfasst die Kapillarstruktur des Stechelements eine zweidimensionale flächige Matrixstruktur mit einer Mehrzahl von Zellen. Sie erstreckt sich insbesondere in dem Flüssigkeitstransferbereich des Stechelementes, in dem der Fluidkontakt mit dem Testfeld stattfindet. Jede der Zellen der Matrixstruktur weist jeweils einen Hohlraum auf, wobei die größte Dimension der Zellenhohlräume der Matrixstruktur geringer ist als die durchschnittliche Breite des Kapillarkanals des Stechelements. Die Zellhohlräume haben eine senkrecht zu der zweidimensionalen Ausdehnung der Matrixstruktur (Matrixebene) definierte Höhe, durch die der Mindestwert der Schichtdicke der Flüssigkeitsschicht gewährleistet ist.

Die Zellenhohlräume der Zellen stehen durch Flüssigkeitspassagen derart in Fluidverbindung miteinander, dass eine in die Matrixstruktur eindringende Flüssigkeit auf eine Mehrzahl von Zellen zweidimensional verteilt wird. Die Zellen sind an einer Übertragungsseite der Matrixstruktur offen, die an die Flüssigkeitseintrittsseite des Testfelds angrenzt. Dadurch kann eine Übertragung der Flüssigkeit aus der Matrixstruktur auf das Testfeld stattfinden.

Als zweidimensionale (flächige) Matrixstruktur wird eine Struktur bezeichnet, bei der eine Mehrzahl von Zellen in einer Matrixebene nebeneinander angeordnet ist. Vorzugsweise sind in der Raumrichtung senkrecht zu der Matrixebene (die parallel zu der Testfeldebene verläuft) nur Zellen in einer Ebene vorhanden. Die flächige Matrixstruktur hat also eine "Höhe" von einer Zelle. Eine von dem Kapillarkanal auf das Testfeld übertragene Flüssigkeit passiert folglich nur eine Zellenhöhe (in Richtung quer zur Ebene der Matrix).

Die flächige Matrixstruktur steht bevorzugt mit dem Kapillarkanal und der Kapillarstruktur in Fluidverbindung und verteilt die von dem Kapillarkanal in die Matrixstruktur strömende Flüssigkeit auf eine im Vergleich mit dem Kapillarkanal größere Breite, bevor die Flüssigkeit die Testschicht des Testfeldes kontaktiert. Es findet eine flächige Verteilung der Flüssigkeit derart statt, dass die Schichtdicke der Flüssigkeit in den Zellen einen gewünschten Mindestwert aufweist. Auf diese Weise wird sichergestellt, dass der von einer Optik erfasste Bereich des Testfelds möglichst gleichmäßig benetzt ist.

Im Rahmen der Erfindung wurde festgestellt, dass mit einer derartigen Zell-Matrixstruktur eine sehr homogene. Verteilung der Flüssigkeit erfolgt. Die Kapillarstruktur hat die Funktion einer Spreitschicht. Wenn die Matrixstruktur netzartig aufgebaut ist, wird in Fachkreisen von einem Spreitnetz gesprochen. Unter Spreitung wird im Sinne der Erfindung die Verteilung einer Flüssigkeit in der Matrixebene derart verstanden, dass ein vordefinierter Mindestwert der Flüssigkeitsschichtdicke in dem Bereich der Benetzung erhalten bleibt und während der Messung an dem Testfeld zur Verfügung steht. Die Flüssigkeit wird in der Matrixstruktur bevorzugt so verteilt, dass eine Mindestfüllhöhe in allen (benetzten) Zellen erhalten bleibt und eine weitere Zelle erst dann gefüllt wird, wenn die Mindestflüssigkeitshöhe in den anderen schon gefüllten Zellen überschritten ist.

Im Rahmen der Erfindung wurde festgesfellt, dass insbesondere bei kleinem Blutvolumen (beispielsweise 100 nl oder weniger) die Art der Blutverteilung auf die einzelnen Zellen der Matrixstruktur von besonderer Bedeutung ist. Die einzelnen Zellen müssen eine definierte Mindestflüssigkeitsdicke aufweisen, damit die Genauigkeit der Messung an der verteilten Flüssigkeit von der Blutmenge (Volumen) bzw. der Höhe der Blutschicht unabhängig ist. Diese Forderung wird von der Zell-Matrixstruktur zuverlässig erfüllt. Das erfindungsgemäße Stechelement ermöglicht darüber hinaus eine sehr kontrollierte und schnelle Spreitung so, dass die Testschicht von den gefüllten Zellen gleichzeitig oder quasi-gleichzeitig benetzt wird. Eine zeitlich sehr unterschiedliche Benetzung führt zu nicht vernachlässigbaren Fehlern bei der Messung. Der Begriff "gleichzeitig" ist in Abhängigkeit von der Messzeit zu betrachten. Die Verteilung gilt als gleichzeitig, wenn die Flüssigkeit innerhalb eines Zehntels, bevorzugt innerhalb eines Zwanzigstels der Messzeit auf die Zellen verteilt wird.

In einer bevorzugten Ausführungsform wird das Stechelement mit dem Kapillarkanal und der Matrixstruktur in einem Verfahrensgang hergestellt. Die tierstillung erfolgt bevorzugt durch Ätzen und/oder durch Laserschneiden.

Das Verfahren zur Herstellung eines Stecheiementes mit einem Kapillarkanal und einer Matrixstruktur ist insbesondere ein Fotoätzverfahren, bei dem nach dem Maskieren mit Fotolack und einer Belichtung aus den maskierten Strukturen mittels Ätzen das Stechelement mit dem Kapillarkanal entsteht und die Matrixstruktur mit einer Mehrzahl von Zellen erzeugt wird.

In einer bevorzugten Ausführungsform wird die zweidimensionale Matrixstruktur durch Ätzen im gleichen Ätzvorgang wie das Herstellen des Kapillar kanals erzeugt. Das ebenfalls bevorzugte Laserschneiden der flächigen Matrixstruktur in einem zusätzlichen Prozessschritt des Lasercuttings erhöht die Herstellungskosten des Testelementes praktisch nicht, da die Investitionskosten des Lasers gering sind.

Die Erfindung wird nachfolgend anhand von den in den Figuren dargestellten bevorzugten Ausführungsformen näher erläutert. Die dort dargestellten Besonderheiten können einzeln oder in Kombination verwendet werden, um bevorzugte Ausgestaltungen der Erfindung zu schaffen. Die hier beschriebenen Ausführungsformen stellen keine Einschränkung der durch die Ansprüche definierten Erfindung dar. Es zeigen:
- Fig. 1: eine Prinzipsskizze eines Analysegeräts mit einer Mehrzahl von Testelementen;
- Fig. 2: eine Ausführungsform eines Testelements;
- Fig. 3 a bis c: Detaildarstellungen alternativer Ausführungsform eines Stechelements mit einer Matrixstruktur;
- Fig. 4 a bis d: eine weitere Ausführungsform eines Stechelements;
- Fig. 5 a bis d: zwei Ausführungsformen einer Kapillarstruktur als Teil des erfindungsgemäßen Testelements;
- Fig. 6 a bis c: alternative Ausführungsformen eines Testelements;
- Fig. 7 a, b: eine Prinzipskizze eines auf einem Bandfolie angeordneten Testelements; und
- Fig. 8: eine Prinzipskizze einer Herstellungsanlage zur Herstellung eines Stechelements.

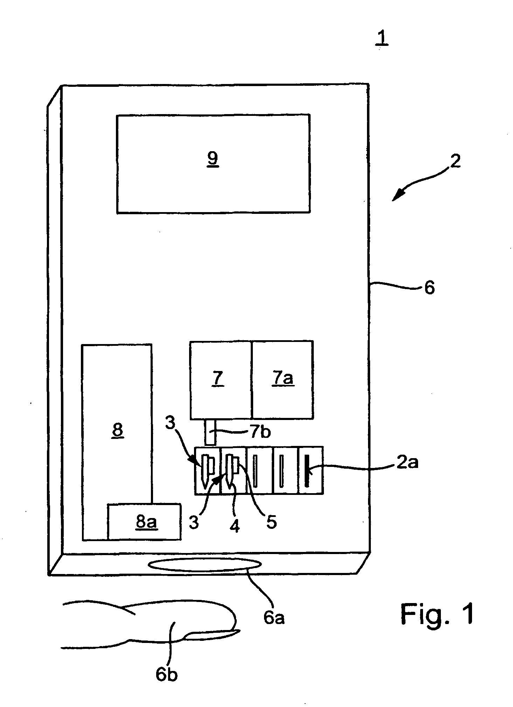

Figur 1 zeigt ein Analysesystem 1 mit einem Analysegerät 2 und einem in dem Gerät 2 enthaltenen Magazin 2a mit Testelementen 3. Die Testelemente 3 umfassen je ein Stechelement 4 und je ein Testfeld 5.

Das Analysegerät 2 hat ein Gehäuse 6, einen Kopplungsmechanismus 7 zur Ankopplung an das Stechelement 4, um es auf einem Bewegungsweg einer Einstichrichtung zu bewegen. Der Kopplungsmechanismus 7 ist mit einer Antriebseinheit 7a gekoppelt, die eine an das Stechelement 4 ankoppelbare Kopplungsstruktur 7b bewegt.

Eine Mess- und Auswerteeinheit 8 dient der Messung einer Änderung eines mit der Körperflüssigkeit reagierenden Reagenz des Analyseelements 5, um einen Analyten der Körperflüssigkeit zu analysieren. Es kann sowohl eine quantitative als auch eine qualitative Analyse vorgenommen werden. Bevorzugt wird der Glukosegehalt im Blut untersucht. In dem gezeigten Beispiel arbeitet die Mess- und Auswerteeinheit 8 nach einem photometrischen Messprinzip und hat eine Optik 8a, die das Testelement 3 vermisst.

Das Analysegerät 2 umfasst eine Anzeigeeinheit (Display) 9 zur Darstellung der Ergebnisse der Analyse. Der Patient oder Benutzer, der das Analysegerät 2 bedient, kann das Resultat direkt abgelesen. Damit ergibt sich für den Patienten oder Benutzer des Analysegerätes 2 ein "one-step-treatment". Er hält das Analysegerät 2 mit einer Anlageöffnung 6a des Geräts 2 derart an seine Fingerbeere 6b, dass das Stechelement 4 eine Einstichwunde in der Fingerbeere 6b erzeugen kann.

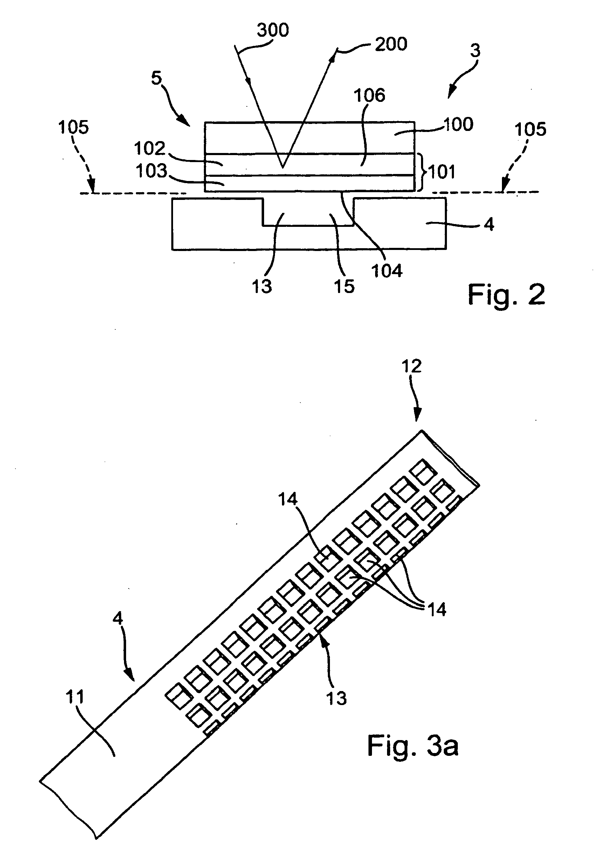

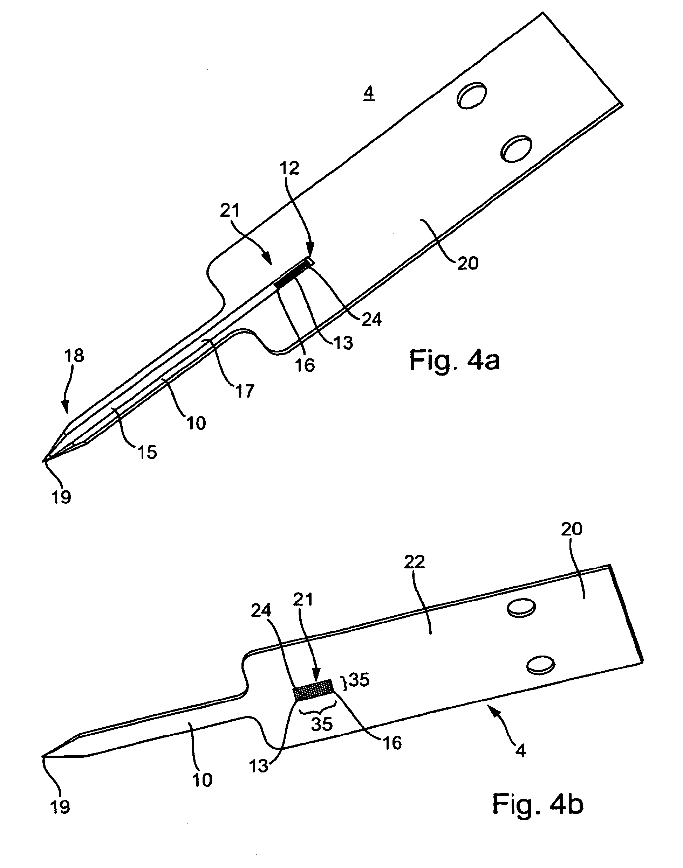

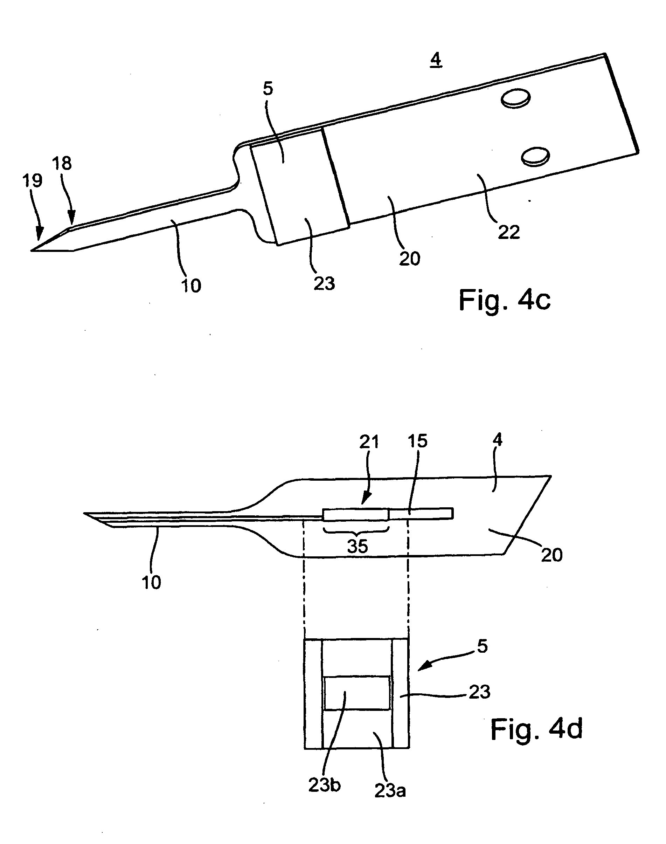

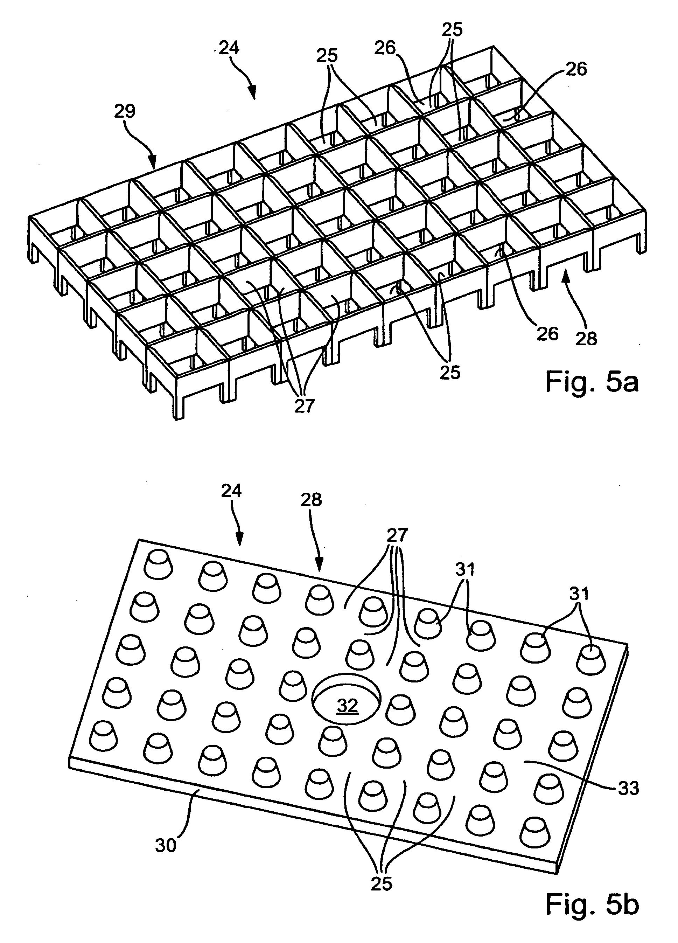

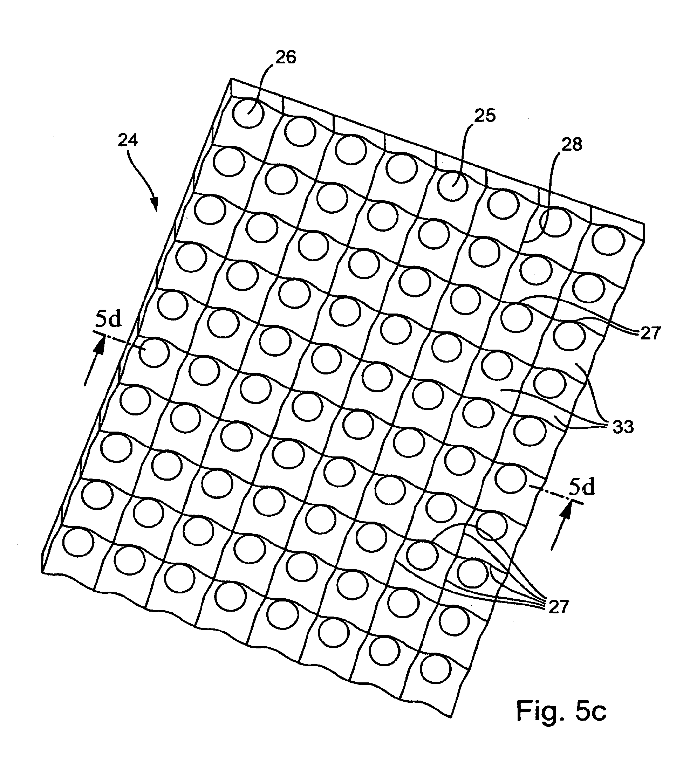

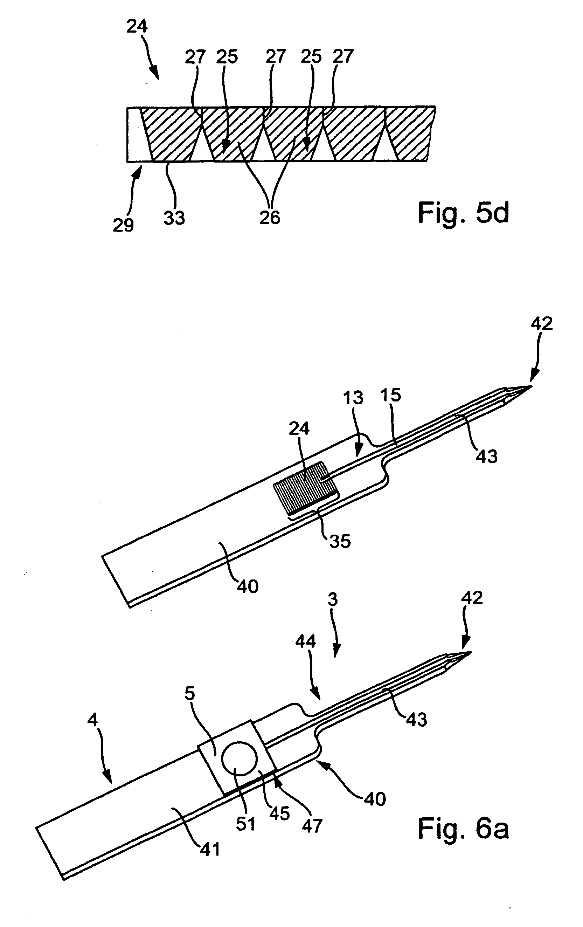

Figur 2 zeigt eine Schnittzeichnung durch ein erfindungsgemäßes Testelement 3 mit einem Stechelement 4 und einem Testfeld 5. Das Stechelement 4 hat eine Kapillarstruktur 13, die im einfachsten Fall von einem Kapillarkanal 15 gebildet wird. Alternativ umfasst die Kapillarstruktur 13 eine Matrixstruktur, wie sie z.B. weiter unten anhand der Figuren 4 und 5 näher erläutert wird.

Das Testfeld 5 umfasst eine transparente flüssigkeitsundurchlässige Tragschicht 100 und eine an die Tragschicht 100 angrenzende Testschicht 101, die auf die Tragschicht 100 beschichtet ist. Im vorliegenden Fall wird die Testschicht 101 aus einer Reaktionsschicht 102 und einer Lichtblockierungsschicht 103 gebildet, wobei die Reaktionsschicht 102 an die transparente Tragschicht 100 angrenzt. Die Lichtblockierungsschicht 103 weist an ihrer der Reaktionsschicht 102 abgewandten Seite eine Flüssigkeitseintrittsseite 104 auf, die eine Testfeldebene 105 bildet. Die Testfeldebene 105 verläuft senkrecht zu der Schnittebene. Sie grenzt an den Kapillarkanal 15 des Stechelements 3 an.

Wie erwähnt, ist die Testschicht 101 mit ihren beiden Teilschichten (Reaktionsschicht 102 und Lichtblockierungsschicht 103) vorzugsweise so ausgebildet, dass durch die Lichtblockierungsschicht die Erythrozyten beim Eindringen der Probe abgetrennt werden, so dass die rote Blutfarbe nicht in einem praktisch störenden Ausmaß in die Reaktionsschicht eindringen kann. Die Reaktionsschicht ist dabei so ausgebildet, dass sie das Licht in nassem Zustand wesentlich weniger streut als die Lichtblockierungsschicht. Beide Schichten sind vorzugsweise auf Basis einer Dispersion oder Emulsion hergestellt, wobei der gleiche Filmbildner verwendet werden kann, jedoch auch die Verwendung unterschiedlicher Filmbildner möglich ist. Weiter ist es bevorzugt, dass zumindest die Reaktionsschicht, vorzugsweise auch die Lichtblockierungsschicht ein Quellmittel enthält. Die Lichtblockierungsschicht enthält darüber hinaus einen Füllstoff, welcher an der Reaktion nicht beteiligt ist, aber das Eindringen der Probenflüssigkeit in die Schicht erleichtert. Im Rahmen der Erfindung wird hierfür vorzugsweise gefällte Kieselsäure (englisch: percipitated silicic acid) verwendet. Die Lichtblockierungsschicht enthält ein stark streuendes Pigment, beispielsweise TiO₂. Jedenfalls werden in den beiden Schichten unterschiedliche Füllstoffe bzw. Pigmente derart eingesetzt, dass die Reaktionsschicht das Licht im nassen Zustand weniger streut, als die Lichtblockierungsschicht. Vorzugsweise ist der Streukoeffizient der Lichtblockierungsschicht mindestens zehnmal so hoch wie der Streukoeffizient der Reaktionsschicht.

Aus Gründen der Übersichtlichkeit entsprechen die in Figur 2 dargestellten Größenverhältnisse des Testelements 3 nicht den realen Verhältnissen. In der Realität ist die Dicke der Tragschicht 100 deutlich größer als die Dicke der durch Beschichten aufgebrachten Testschicht 101. Die Tragschichtdicke beträgt bevorzugt ca. 50 µm bis ca. 200 µm. Die Dicke der Testschicht 101 beträgt nur wenige µm, bevorzugt weniger als 30 µm, besonders bevorzugt weniger als 20 µm und ganz besonders bevorzugt weniger als 10 µm. Bevorzugt liegt das Verhältnis der Dicken der Lichtblockierungsschicht 103 und der Reaktionsschicht 102 zueinander in einem Bereich von 3:1 bis 1:3. Das Testfeld 5 grenzt derart an das Stechelement 4 an und kontaktiert es in der Regel, dass die Testfeldebene 105 bündig mit der Flüssigkeitseintrittsseite 104 und mit der Oberseite des Stechelements 4 sowie der offenen Seite des Kapillarkanals 15 ist. Die Testfeldebene 105 entspricht somit der Übertragungsseite der Kapillarstruktur 13.

In den Kapillarkanal 13 eintretende Flüssigkeit benetzt die Testschicht 101, so dass Flüssigkeit in die Testschicht 101 eindringt. Eine optische Messung der durch die Flüssigkeit in der Testschicht 101 hervorgerufenen Farbänderung geschieht durch einen Lichtstrahl 200, der an der der Testschicht 101 abgewandten Seite der Tragschicht 100 in die Tragschicht 100 eindringt. Der Lichtstrahl 200 wird innerhalb der Testschicht 101, bevorzugt innerhalb der Reaktionsschicht 102 der Testschicht 101 gestreut und reflektiert. Die Reaktionsschicht 102 ist eine Detektionsschicht 106, in der die Farbänderung auftritt. Durch die während der Reaktion in der Testschicht 101 ablaufenden Farbänderung ändert sich die Streuung und damit der von dem Testelement 3 reflektierte Lichtstrahl, der optisch ausgewertet werden kann. Die Lichtblockierungsschicht 103 verhindert, dass das Licht 200 in die Kapillarstruktur 13 eindringt und beispielsweise rote Blutkörper von in dem Kapillarkanal vorhandenen Blut die optische Messung beeinflussen.

Figur 3a zeigt einen Ausschnitt einer besonderen Ausführungsform eines Stechelements 4, das eine Hohlnadel 11 mit einer hier nicht gezeigten Spitze ist. Die Hohlnadel 11 weist eine Kapillarstruktur 13 auf, die an einem der Spitze gegenüberliegenden fernen Ende 12 der Hohlnadel 11 mehrere, in die Au-βenwand der Hohlnadel 11 integrierte, nebeneinander angeordnete Ausnehmungen 14 umfasst. Die Ausnehmungen 14 können beispielsweise quadratisch sein, wobei die Dimension der Ausnehmungen 14 in Umfangsrichtung höchstens 50 µm, bevorzugt höchstens 30 µm beträgt. Die Höhe der Ausnehmungen 14, also die Dimension in radialer Richtung der Hohlnadel 11, beträgt vorzugsweise zwischen 30 µm und 100 µm, bevorzugt mindestens 50 µm. Sie ist durch die Materialstärke der Hohlnadel 11 bestimmt. Da die Dimensionen der Ausnehmungen 14 kleiner sind als der Durchmesser der Hohlnadel 11, ist die Kapillarwirkung in die einzelnen Ausnehmungen 14 größer als die der Hohlnadel 11. Das Blut strömt aus der Nadel 11 in die Ausnehmungen 14 und verteilt sich gleichmäßig auf mehrere Ausnehmungen 14. Bei Annäherung eines mit der Hohlnadel 11 korrespondierenden, bevorzugt gebogenen, Testfelds 5 bildet die Kapillarstruktur 13 eine Flüssigkeitshaltestruktur für das Testfeld 5, das selbst keine eigene Flüssigkeitshaltestruktur aufweist.

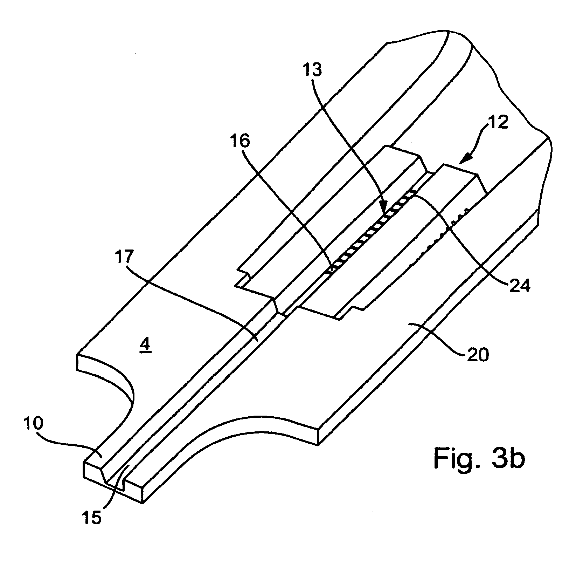

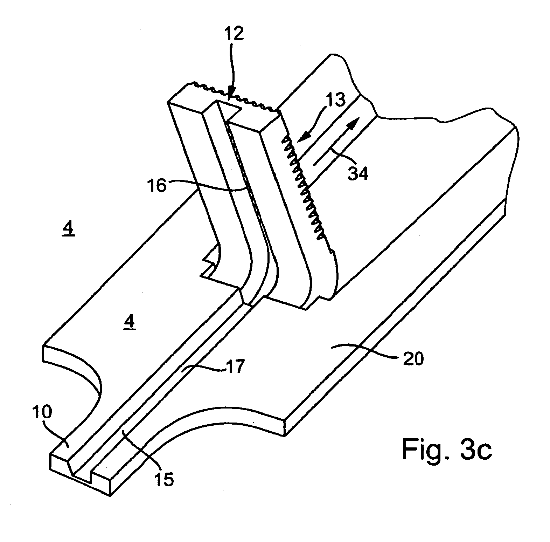

Die Figuren 3b und 3c zeigen je einen Ausschnitt von zwei weiteren Ausführungsformen eines erfindungsgemäßen Stechelements 4. Es ist als flaches Nadelelement 10 ausgebildet und weist eine Kapillarstruktur 13 auf, die einen einseitig offenen Kapillarkanal 15 umfasst, der sich von einer hier nicht dargestellten Spitze weg erstreckt. Der Kapillarkanal 15 ist an seinem fernen Ende 12 beidseitig offen, wobei sich eine zweite Öffnung 16 am Boden 17 des Kapillarkanals 15 erstreckt. Zusätzlich ist der Kapillarkanal 15 an seinem Ende 12 ebenfalls offen. Die zweite Öffnung 16 am Boden 17 ist ebenfalls Teil der Kapillarstruktur 13 und kann z.B. in Form eines Netzes oder einer Matrixstruktur ausgebildet sein.

Figur 3c zeigt eine Ausführungsform, bei der das ferne Ende 12 des Kapillar kanals 15 (bevorzugt um 90 Grad) abgewinkelt ist. Durch eine Relativbewegung in Richtung des Pfeiles 34 zwischen dem Stechelement 4 und einem hier nicht dargestellten Testfeld kann die Kapillarstruktur 13 zur Übertragung einer Probenflüssigkeit in Fluidkontakt mit dem Testfeld (Flüssigkeitstransferposition) gebracht werden. Die Kapillarstruktur 13 bildet dann die Flüssigkeitshaltestruktur des Analyseelements.

In den Figuren 4a bis 4d ist eine weitere Ausführungsform eines erfindungsgemäßen Testelements 3 gezeigt, das ein ebenfalls flaches Stechelement 4 hat, an dessen einem Ende ein Nadelelement 10 ausgebildet ist. Das Nadelelement 10 hat an seinem freien Ende 18 eine Spitze 19 zum Erzeugen einer Einstichwunde. Von der Spitze 19 erstreckt sich ein einseitig offener Kapillarkanal 15 in Längsrichtung des Nadelelementes 10 bis in einen flachen Stechkörper 20 des Stechelements 4 hinein. In einem dem fernen Ende 12 des Kapillarkanals 15 nahen Bereich 21 hat der Kapillarkanal 15 in seinem Boden 17 eine weitere Ausnehmung 16; er ist folglich beidseitig offen.

Der beidseitig offene Bereich 21 des Kapillarkanals 15 ist ein von der Spitze 19 abgewandter Abschnitt des Kapillarkanals 15, der näher bei dem fernen Ende 12 als bei der Spitze 19 angeordnet ist. Unterhalb des Bereichs 21 ist eine kapillare Struktur, insbesondere eine Matrixstruktur 24 angeordnet, die einströmende Flüssigkeit verteilt. Der Kapillarkanal 15 kann sich über diesen Abschnitt hinaus erstrecken. Dies hat den Vorteil, dass von der Spitze 19 in den Kapillarkanal 15 eintretendes Blut zuerst über die Matrixstruktur 24 hinwegströmt, so dass sich eine erste Teilmenge des Bluts in einem am fernen Ende 12 des Kapillarkanals 15 angeordneten Abschnitt ansammelt. Diese Teilmenge des Bluts dringt nicht in die Matrixstruktur 24 ein, da die Fließgeschwindigkeit innerhalb des Kapillarkanals 15 so groß ist, dass die Kapillarwirkung der Struktur 24 der von der Fließgeschwindigkeit ausgeübten Kraft unterliegt. Auf diese Weise wird verhindert, dass die erste Teilmenge des Bluts analysiert wird, die beispielsweise durch Schweißpartikel verunreinigt sein kann. Sobald das Blut bis an das freie Ende des Kapillarkanals 15 gelangt ist, sinkt die Fließgeschwindigkeit. Die Kapillarwirkung der Struktur 24 saugt nun das Blut ein.

Figur 4b zeigt die Unterseite 22 des Stechelements 4. In dem offenen Bereich 21 ist die als Matrixstruktur 24 ausgebildete Kapillarstruktur 13 angeordnet. Sie ist bevorzugt einstückig mit dem Stechelement 4 verbunden. Besonders bevorzugt werden Stechelement 4 und Matrixstruktur 24 aus einem Element gebildet. Sie sind beide aus Metall. Der offene Bereich 21 ist ein Flüssigkeitstransferbereich 35, in dem die Übertragung einer Flüssigkeit von der Kapillarstruktur 13 (über die Matrixstruktur 24) auf ein benachbartes Testfeld 5 (Fig. 4c) stattfindet.

Figur 4c zeigt, dass ein Testfeld 5 auf der Unterseite 22 des Stechelements 4 angeordnet ist, wobei die Kapillarstruktur 13 von dem Testfeld 5 abgedeckt wird. Das Testfeld 5 umfasst eine Tragschicht 23 und eine Testschicht 23a mit einer Testzone 23b, Figur 4d. Bevorzugt ist die Testzone 23b an den offenen Bereich 21 und die dort angeordnete Kapillarstruktur 13 angepasst. Der Flüssigkeitstransferbereich 25 erstreckt sich über die hier nicht dargestellt Matrixstruktur 24. Der Flüssigkeitstransferbereich 35 ist in dieser Ausführungsform geringfügig kleiner ausgebildet als die Testzone 23b; er kann aber auch größer oder gleich groß sein wie die Testzone 23b. In dieser Ausführungsform entspricht der Flüssigkeitstransferbereich 35 mindestens dem offenen Bereich 21.

Die Figuren 5a, 5b und 5c, 5d zeigen verschiedene Ausführungsformen einer zweidimensionalen Matrixstruktur 24, die Teil einer Kapillarstruktur 13 eines erfindungsgemäßen Testelements 3 sein können. Die zweidimensionale Matrixstruktur 24 hat eine Mehrzahl von Zellen 25, die jeweils einen Zellenhohlraum 26 aufweisen, dessen größte Dimension kleiner ist als die durchschnittliche Breite des Kapillarkanals des Testelements 3. Aufgrund der geringeren Dimensionen der Zellen 25 ist die Kapillarwirkung der Matrixstruktur 24 größer als die des Kapillarkanals 15, so dass Flüssigkeit aus dem Kapillarkanal 15 in die Matrixstruktur 24 einströmt. Die auftretende Kapillarkraft ist dabei die wesentliche, bevorzugt die ausschließliche Komponente. Die Dicke der Flüssigkeitsschicht und das damit im Zusammenhang stehende Flüssigkeitsvolumen in der Matrixstruktur 24 ist kleiner als das Volumen, das durch den Flüssigkeitskanal (Kapillarkanal 15) angeboten wird und für eine Messung zur Verfügung steht. Damit wird erreicht, dass die Matrixstruktur 24 stets hinreichend gefüllt ist und das Messergebnis von der primär zur Verfügung stehenden Flüssigkeitsmenge unabhängig ist.

In diesen bevorzugten Ausführungsformen stehen die Zellhohlräume 26 der Zellen 25 durch Flüssigkeitspassagen 27 derart in Fluidverbindung miteinander, dass eine in die Matrixstruktur 24 eindringende Flüssigkeit auf eine Mehrzahl von Zellen 25 zweidimensional verteilt wird. Die Zellen 25 sind zur Übertragung der Flüssigkeit auf eine Testschicht des Testfelds 5 an einer Übertragungsseite 28 der Matrixstruktur 24 offen.

Die Matrixstruktur 24 gemäß Figur 5a weist die Form eines Gitterrostes auf, das an einer Oberseite offen ist. Die Oberseite ist bevorzugt eine Flüssigkeitseintrittsseite 29. Die Zellen 25 haben einen rechteckigen, bevorzugt einen quadratischen Querschnitt. Die Seitenlänge der Zellen sollte 20 bis 50 µm betragen. Ihre Dicke beträgt vorzugsweise 20 bis 100 µm. Sie können selbstverständlich auch beliebige Formen (rund) annehmen.

Bei einer Anordnung der zweidimensionalen Matrixstruktur 24 parallel und benachbart zu einem Kapillarkanal 15 eines Stechelements 4 findet ein Flüssigkeitsübertrag von dem Kapillarkanal 15 auf die Matrixstruktur 24 statt. Die als Unterseite ausgebildete Übertragungsseite 28 der Matrixstruktur 24 ist ebenfalls offen, so dass die Flüssigkeit in das Testfeld 5 übertragen werden kann. Dabei wird die Flüssigkeit in der Matrixstruktur 24 so verteilt, dass in den einzelnen Zellen 25 eine gewünschte Flüssigkeitssäule entsteht.

Unter dem Begriff zweidimensionale (flächige) Matrixstruktur 24 wird eine Struktur derart verstanden, dass die Zellen 25 jeweils nur in einer Ebene (Matrixebene) benachbart sind. Eine Anordnung mehrerer Zellen 25 übereinander ist nicht vorgesehen.

Wie in Figur 5a gezeigt, kann die Matrixstruktur 24 zu beiden Seiten hin offen sein. So können bei Anordnung der Matrixstruktur 24 am Ende des Kapillarkanals 15 und seitlichem Einfließen der Probenflüssigkeit in die Matrixstruktur 24 sowohl an der Unterseite (Übertragungsseite 28) als auch an der Oberseite der Matrixstruktur 24 ein Testfeld angeordnet sein. Dadurch ist eine Doppelmessung zur unabhängigen Kontrolle der Messergebnisse möglich. Alternativ können auch zwei Messungen gleichzeitig durchgeführt, beispielsweise wenn zwei unterschiedliche Analyten oder unterschiedliche Parameter aus nur einer Probe bestimmt werden sollen. Es hat sich als vorteilhaft erwiesen, wenn die Matrixstruktur 24, wie in Fig. 5a, aus Metall besteht, da die Benetzung einer Metallstruktur deutlich schneller erfolgt als die Benetzung eines Gewebes oder Vliesmaterials.

Die Ausführungsform der Matrixstruktur 24 gemäß Figur 5b zeigt einen plattenförmigen Grundkörper 30, an dessen Unterseite eine Mehrzahl von Noppen 31 angeordnet ist. Zwischen den Noppen 31 werden die einzelnen Zellen 25 der Matrixstruktur 24 gebildet. Der Grundkörper 30 hat eine Ausnehmung 32 an seiner Oberseite, durch die Flüssigkeit in die einzelnen Zellen 25 gelangen kann. Eine Flüssigkeit wird auf eine Mehrzahl von Zellen 25 verteilt, wobei in jeder der mit Flüssigkeit gefüllten Zellen 25 eine vorgegebene Schichtdicke der Flüssigkeit (Flüssigkeitssäule) entsteht und eine Weiterverteilung auf benachbarte Zellen 25 erst danach stattfindet. Die vorgegebene Flüssigkeitssäule in den zuerst gefüllten Zellen 25 bleibt erhalten.

Die Übertragung der Flüssigkeit von der Matrixstruktur 24 auf ein an die Struktur 24 angrenzendes Testfeld 5 findet über die offene Noppenseite (Übertragungsseite 28) der Matrixstruktur 24 statt, an der die Zellen 25 Zellöffnungen 33 aufweisen, durch die die Flüssigkeit austritt.

Die Figuren 5c und 5d (Schnittdarstellung) zeigen eine weitere Ausführungsform einer Matrixstruktur 24, deren Zellen 25 derart ausgebildet sind, dass sich bevorzugt die Zellhohlräume 26 zur Übertragungsseite 28 hin erweitern. Vorzugsweise erweitern sich die Zellhohlräume 26 stetig. Die Matrixstruktur 24 ist an ihrer Oberseite (Flüssigkeitseintrittsseite 29) derart offen, dass eine Flüssigkeit in die einzelnen Zellen 25 eindringen kann. An der Unterseite (Übertragungsseite 28) weisen die Zellen 25 Zellöffnungen 33 zur Übertragung der Flüssigkeit auf eine benachbarte Testschicht auf. Die einzelnen Zellen 25 stehen über Flüssigkeitspassagen 27 derart in Fluidverbindung miteinander, dass die Flüssigkeit auf mehrere Zellen 25 verteilt wird.

Die Höhe der Zellen 25 ist in einer bevorzugten Ausführungsform zwischen 20 µm und 150 µm groß. Vorteilhafterweise liegt die Zellhöhe zwischen 30 µm und 100 µm. Besonders bevorzugt ist sie mindestens 50 µm groß. Derartige Zellhöhen ermöglichen es, auch kleine Flüssigkeitsvolumina von höchstens 50 nl zu untersuchen. Die Spreitschicht (Matrixstruktur 24) nach Figur 5c hat eine Größe von 320 µm x 400 µm, die sich aus den 80 Zellen 25 mit quadratischem Querschnitt berechnet. Die Zellen 25 weisen jeweils eine Seitenkante von 40 µm und eine Höhe von 60 µm auf. Jede Zelle 25 hat somit ein Bruttovolumen von 0,096 nl. Das Gesamtvolumen der Matrixstruktur 24 beträgt etwa 7,7 nl. Da die Spreitschicht ein effektives Flüssigkeitsaufnahmevolumen von ca. 60 % des Gesamtvolumens hat, kann in ihr ein Volumen von ca. 4,5 nl aufgenommen werden. Dabei wird die Flüssigkeit auf eine Mehrzahl von Zeilen 25 (bevorzugt auf alle Zellen 25) mit einer hinreichenden Flüssigkeitssäule (mindestens 50 µm) oberhalb der angrenzenden Testschicht verteilt, so dass die optische Messung unabhängig von der Flüssigkeitsmenge ist.

In einer bevorzugten Ausführungsform weist die Matrixstruktur 24 wenigstens 20 Zellen 25, bevorzugt wenigstens 25 Zellen 25 und besonders bevorzugt wenigstens 50 Zellen 25 auf. Noch weiter bevorzugt ist eine Ausführungsform bei der die Matrixstruktur 24 wenigstens 100 Zellen 25 umfasst.

Die große Anzahl von Zellen 25 im Zusammenhang mit dünnen Zellwänden, die im Vergleich zu den Abmessungen der Zellen 25 sehr klein (Zellwanddicke kleiner 10 µm) sind, ermöglicht eine statistische Auswertung bei einer optischen Vermessung der Matrixstruktur 24. Da an den Schnittpunkten der Zellenwände der Zellen 25 keine hinreichende Benetzung bzw. Bedeckung der Matrixstruktur 24 mit Flüssigkeit stattfindet, ist an diesen Stellen keine optische Auswertung möglich. Erst durch die Verteilung auf eine Mehrzahl von Zellen 25 mit einer hinreichenden Flüssigkeitssäule wird sichergestellt, dass genügend viele Zellen 25 und die darin enthaltenen Flüssigkeitsvolumina in die optische Ausmessung einfließen. Bei einer genügend hohen Anzahl von Zellen 25 (mindestens 20, bevorzugt mindestens 50) kann durch eine statistische Auswertung der Einfluss der Zellwände und der Knotenpunkte eliminiert werden.

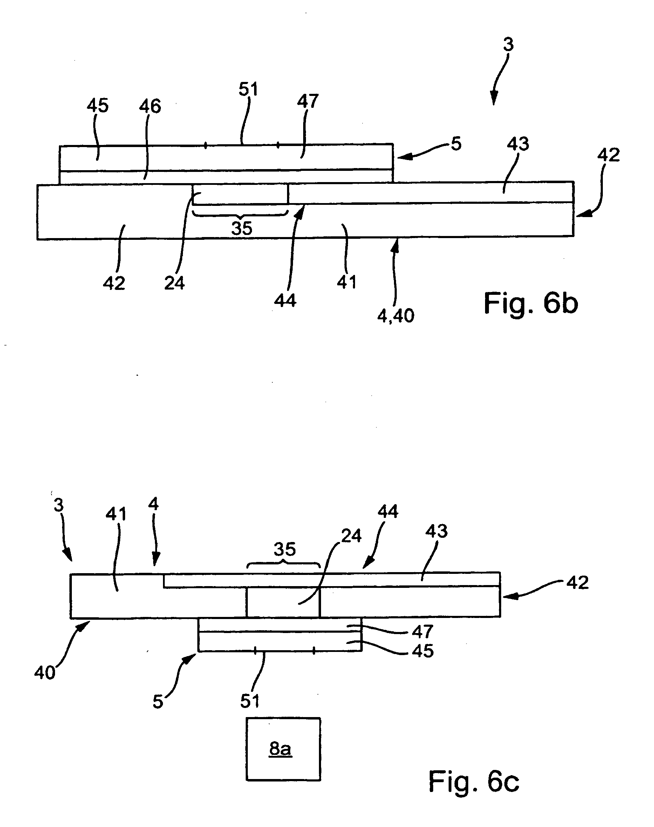

Die Figuren 6a bis 6c zeigen ein erfindungsgemäßes Testelement 3, mit einem Stechelement 4 und einem Testfeld 5. Figur 6a zeigt das Testelement 3 mit Testfeld 5 (unten) und ohne Testfeld 5 (oben). Das Stechelement 4 ist eine Lanzette 40, deren flacher Lanzettenkörper 41 sich zu einem Ende hin in eine Spitze 42 verjüngt. Eine Kapillarstruktur 44 umfasst einen Kapillarkanal 43 und eine zellenartige Struktur mit Kapillarwirkung, z. B. eine Matrixstruktur 24 oder eine Zellenstruktur. Ein Kapillarkanal 43 erstreckt sich von der Spitze 42 in Richtung Lanzettenkörper 41 zu einem Flüssigkeitstransferbereich 35, in dem eine Matrixstruktur 24, die Teil der Kapillarstruktur 44 ist, angeordnet ist. In Figur 6c erstreckt sich der Kapillarkanal 43 auch über den Flüssigkeitstransferbereich 35 hinaus. Benachbart zu der Matrixstruktur 24 ist ein Testfeld 5 positioniert, das aus einer Tragschicht 45 und einer Testschicht 47 besteht. Das Testfeld 5 hat keine eigene Flüssigkeitshaltestruktur.

Das Testelement 3 eignet sich durch die optisch homogene Verteilung einer Flüssigkeit besonders für eine photometrische Messung einer charakteristischen Messgröße. Die Tragschicht 45 ist transparent, so dass das Testfeld 5 von einer Optik 8a (vgl. Fig. 6c) optisch erfasst und ausgewertet werden kann. Da die Matrixstruktur 24 die Flüssigkeit aufspreitet, kann eine konventionelle, preiswerte Optik zur photometrischen Messung verwendet werden, die zum Beispiel auf 50 µm fokussiert. Produktions- und Lagetoleranzen, die sich auf die Relativposition zwischen Optik 8a und dessen Beleuchtungsfleck 51 zu der Testschicht 47 auswirken, spielen keine Rolle, da sie größer als der Beleuchtungsfleck 51 (optischer Auswertebereich) ist.

Figur 6c zeigt eine Ausführungsform des Testelements 3, bei der das Testfeld 46 an der dem Kapillarkanal 43 gegenüberliegenden Seite der Lanzette 40 angeordnet ist. Die Matrixstruktur 24 ist parallel zum Kapillarkanal 43 in dem Flüssigkeitstransferbereich 35 angeordnet, der nicht direkt am Ende des Kapillarkanals 43 liegt. Im Gegensatz hierzu ist dem Ausführungsbeispiel nach Figur 6b die Matrixstruktur 24 am Ende des Kapillarkanals 43 angeordnet. In beiden Fällen ist die flächige Matrixstruktur 24 einstückig mit der Lanzette 40 verbunden. Sie wird bevorzugt durch Ätzen im gleichen Arbeitsgang wie die Lanzette 40 hergestellt.

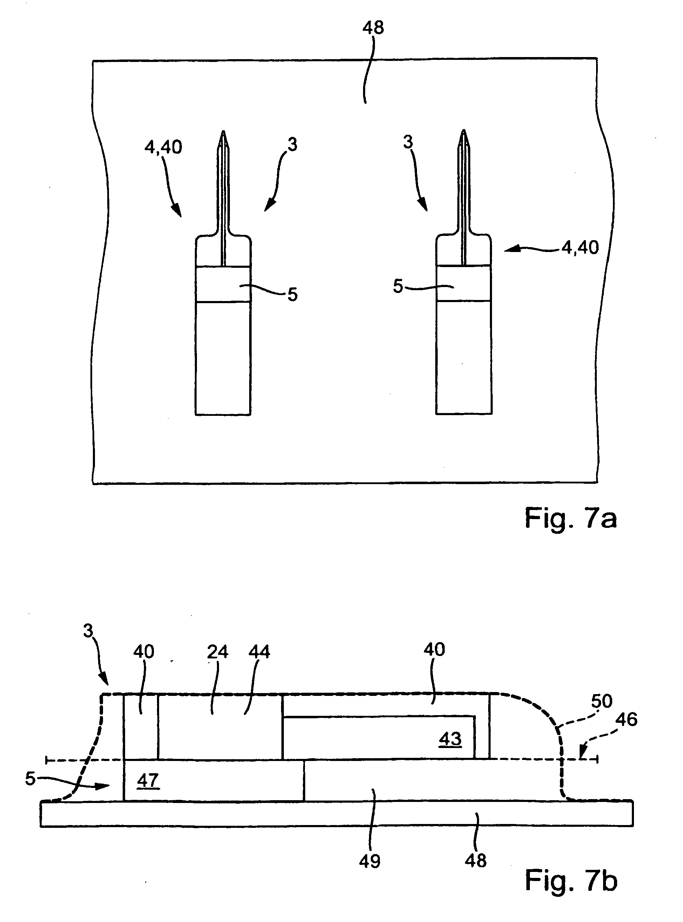

Die Figuren 7a und 7b zeigen das Testelement 3 aus Figur 6a mit Stechelement 4 (Lanzette 40) und Testfeld 5, das als "lancet-on-tape" ausgeführt ist. Die Tragschicht 45 des Testfelds 5 ist als Bandfolie 48 ausgeführt, auf dem mehrere Testelemente 3 in einem vorgegebenen Abstand positioniert sind. Die Testelemente 3 sind an der Unterseite der transparenten Bandfolie 48 aufgeklebt. Die Klebefläche schließt sich vorteilhafterweise an das Testfeld 46 an. Klebende Substanzen können auch in die Testschicht 47 integriert sein.

Figur 7b zeigt einen Schnitt durch das auf der Bandfolie 48 positionierte Testelement 3. Die Bandfolie 48 bildet die Tragschicht des Testfelds 5. Die Lanzette 40 kontaktiert das Testfeld an einer Testfeldebene 46 derart, dass die Flüssigkeitseintrittsseite 29 an die Übertragungsseite 28 der Matrixstruktur 24 angrenzt. Die Lanzette 40 ist mit der Bandfolie 48 nur über die Testschicht 47, alternativ zusätzlich über eine Klebefläche, verbunden. Zwischen der Lanzette 40 (mit ihrem Kapillarkanal 43) und der Bandfolie 48 wird ein Freiraum 49 gebildet, in dem überschüssige Körperflüssigkeit gesammelt werden kann.

Da der größte Teil des Lanzettenkörpers 41, insbesondere der verjüngte Teil des Lanzettenkörpers 41 mit dem Kapillarkanal 43 und der Spitze, nicht an der Bandfolie 48 befestigt ist, kann zum Einstich der Lanzette 40 in ein Körperteil die Bandfolie 48 relativ zu der Lanzette 40 (nach unten) abgeklappt oder abgewinkelt werden, so dass die Spitze 42 in das Körperteil einstechen kann, ohne dass der Einstich von der Bandfolie 48 negativ beeinflusst wird.

Aus Gründen der Sterilhaltung während der Lagerung können die einzelnen Testelemente 3 auf der Bandfolie 48 mit einer Sterilschutzfolie 50 abgedeckt werden. Die Folie 50 kann entweder über Teilbereiche oder die gesamte Bandfolie 48 gespannt sein. Die Bandfolie 48 mit einer Vielzahl von Testelementen 3 kann in einem Magazin oder einer Kassette gelagert werden, insbesondere in einer Kassette aufgerollt werden. Auch bei einem Aufwickeln mit geringem Radius löst sich eine Flüssigkeitshaltestruktur (Matrixstruktur 24) nicht von dem Testfeld 5 ab.

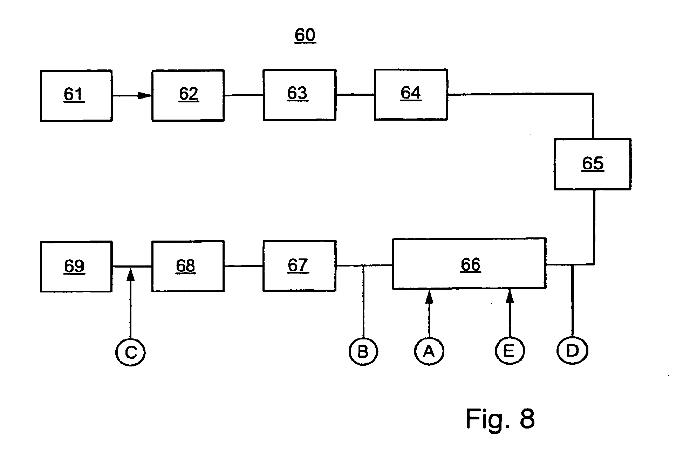

Figur 8 zeigt schematisch eine Herstellungsanlage 60 zum Herstellen eines erfindungsgemäßen Stechelements 4 mit einem Kapillarkanal 15 und einer Kapillarstruktur 13, wie oben beschrieben. Mit der Anlage 60 können im Endlosverfahren auf einem Metallband eine Vielzahl von Stechelementen 4 in Massenfertigung hergestellt werden. Auf einem Abwickler 61 ist ein dünnes Metallband aufgewickelt, das nach dem Abwickeln in einer Reinigungsanlage 62 gereinigt wird. In einer Beschichtungseinheit 63 wird das Metallband bevorzugt beidseitig mit einer lichtempfindlichen Schicht zum Beispiel mit einem flüssigen oder festen Fotolack versehen. In einer Printereinheit 64 wird eine Maske in Form der Kontur des herzustellenden Stechelementes auf das Metallband aufgebracht. Das Metallband wird schließlich belichtet. In einer Entwicklereinheit 65 wird das beschichtete und belichtete Metallband entwickelt.

Die durch die Maske abgedeckten Bereiche werden frei gespült. In einem nächsten Verfahrensschritt werden in einer Ätzeinheit 66 die unbeschichteten Flächen an-, weg- oder durchgeätzt. Hier entstehen wenigstens das Stechelement 4 mit Spitze und der Kapillarkanal 15.

Anschließend wird in einer Entschichtungseinheit 67 die ätzresistente Beschichtung entfernt und das Metallband gereinigt. Nach der Trocknung in einem Trockner 68 wird es in einem Aufwickler 69 wieder auf eine Rolle aufgewickelt. Alternativ könnten die einzelnen Stechelemente 4 auch aus dem Band herausgetrennt werden. Alternativ kann das Metallband mit den Stechelementen auch mit einer Trägerfolie oder Bandfolie, die die Tragschicht der Testelemente bildet, und einer darauf beschichteten Testschicht verbunden werden, so dass ein Band mit erfindungsgemäßen Testelementen entsteht, die in einem weiteren Verfahrensschritt vereinzelt und/oder magaziniert werden können.

In einer bevorzugten Ausführungsform des Herstellungsverfahrens werden in der Ätzeinheit 66 nicht nur der Kapillarkanal 15 und die Kontur des Stechelementes 4 geätzt sondern auch die flächige Matrixstruktur 24 im gleichen Ätzvorgang hergestellt. Mit Hilfe des photochemischen Feinätzens ist es möglich, die Matrixstruktur 24 mit einer Mehrzahl von Zellen 25 herzustellen, wobei die größte Dimension der Zellen 25 zwischen 30 µm und 100 µm ist. Bevorzugt beträgt die Höhe der Zellen, die der Dicke des Metallbandes entspricht, zwischen 45 µm und 100 µm.

In einer ebenfalls bevorzugten Ausführungsform des Herstellungsverfahrens kann die zweidimensionale Matrixstruktur 24 nach dem Ätzvorgang, in dem das Stechelement 4 mit dem Kapillarkanal 15 hergestellt wird, durch Lasern erfolgen. Dazu wird eine Lasereinheit mit einem Laser, beispielsweise einem Excimerlaser, eingesetzt.

Bevorzugt ist die Lasereinheit an den in Figur 8 bezeichnet Stellen B und C angeordnet. Die Positionierung an dem Punkt C hat den Vorteil, dass die unterschiedlichen Verfahren des Ätzens und Lasern räumlich getrennt sind. Jeglicher Einfluss von Ätzmittel auf die gelaserte Struktur wird vermieden. Da das gereinigte Metallband mit dem Stechelementen gelasert wird, entstehen beim Lasern keine Rauchgase aus einem anderen Material wie z.B. Photolack.

In einer ebenfalls bevorzugten Ausführungsform wird das Laserschneiden vor dem Ätzvorgang durchgeführt. Bevorzugte Positionen der Lasereinheit sind an den Stellen D und E in Figur 8 gezeigt. Die bereits gelaserte Struktur wird während des Ätzens entgratet.

Die Lasereinheit kann alternativ an der in Figur 8 mit A bezeichneten Stelle positioniert sein. Das Lasern erfolgt zwischen zwei Ätzvorgängen, insbesondere vor dem letzten Ätzvorgang. Bei dieser Laserposition werden durch den kurzen Einfluss des Ätzmittel die gelaserten Strukturen entgratet, ohne die Strukturen jedoch unzulässig zu verändern oder zu verfälschen.

## Patentansprüche

1. Testelement zum Erzeugen einer Einstichwunde in einem Körperteil, zur Aufnahme einer Körperflüssigkeitsprobe aus dem Körperteil und zur Analyse mittels eines Reagenzsystems, dessen Reaktion mit einem in der Körperflüssigkeit enthaltenen Analysen zu einer Änderung einer an dem Testelement optisch messbaren für das gewünschte analytische Ergebnis charakteristischen Messgröße führt,
umfassend ein Steckelement (4) und ein Testfeld (5), das mindestens einen Teil des Reagenzsystems enthält, wobei
das Stechelement (4) eine an einem Ende des Stechelementes (4) angeordnete Spitze zum Erzeugen einer Wunde in dem Körperteil und eine Kapillarstruktur (13, 44) aufweist, die so ausgebildet ist, dass nach dem Einstechen der Spitze des Stechelementes in die Haut Korperfüssigkeil in die Kapillarstruktur (13, 44) eindringt,
das Stechelement (4) und das Testfeld (5) relativ zueinander so in einer Flüssigkeitstransferposition positionierbar sind, dass das Testfeld in Fluidkontakt zu einem Teil der Kapillarstruktur des Stechelementes steht, wobei in die Kapillarstruktur eingedrungene Körperflüssigkeit zu dem Testfeld transferiert werden kann,
das Testfeld (5) eine transparente, nicht poröse Tragschicht (45, 100) und eine auf der Tragschicht (45, 100) durch Beschichten angebrachte Testschicht (101) umfasst, die nicht in die Tragschicht (45, 100) eindringt, wobei die von der Tragschicht (45, 100) abgewandte Seite der Testschicht (101) die Flüssigkeitseinlrittsseile (104) bildet, die in der Flüssigkeitstransferposition der Kapillarstruktur (13) zugewandt ist und
in der Flüssigkeitstransferposition die Flüssigkeitseintrittsseite (104) der Testschicht (101) unmittelbar an die Kapillarstruktur (13, 44) derart angrenzt, dass ein direkter Flüssigkeitsübertritt von der Kapillarstruktur (13, 44) in die Testschicht (101) stattfindet.

2. Testelement nach einem der vorhergehenden Ansprüche, **dadurch gekennzeichnet, dass** das Testfeld an dem Stechelement fixiert ist, so dass das Stechelement und das Testfeld permanent in FluidkontaKt stehen.

3. Testelement nach einem der vorhergehenden Ansprüche, **dadurch gekennzeichnet, dass** die Testschicht (101) zwei Teilschichten einschließt, wobei eine erste auf die Tragschicht beschichtete Teilschicht eine Reaktionsschicht (102) und eine zweite darauf beschichtete Teilschicht eine opake Lichtblockierungsschicht (103) ist.

4. Testelement nach Anspruch 3, **dadurch gekennzeichnet, dass** sowohl die Reaktionsschicht (102)als auch die opake Lichtblockierungsschicht (103) ein Enzym und ein Farbbildungsreagenz (Indikator) enthalten, wobei die Menge des Enzyms in der Reaktionsschicht (102) höher als in der Lichtblockierungsschicht ist (103) und die Menge des Indikators in der Reaktionsschicht (102) geringer als in der Lichtblockierungsschicht (103) ist.

5. Testelement nach einem der vorhergehenden Ansprüche, **dadurch gekennzeichnet, dass** mindestens ein Bestandteil des Reagenzsystems, vorzugsweise ein Enzym, an eine andere Komponente der Testschicht (191), insbesondere einen in der Testschicht (102) enthaltenen Füllstoff, gebunden ist.

6. Testelement nach einem der vorhergehenden Ansprüche, **dadurch gekennzeichnet, dass** die Kapillarstruktur (13, 44) eine zweidimensionale Matrixstruktur (24) mit einer Mehrzahl von Zellen (25) mit Zellhohlräumen (26) und einen Kapillarkanal einschließt, durch den nach dem Einstechen der Spitze des Stechelementes (4) in die Haut Körperflüssigkeit zu der zweidimensionalen Matrixstruktur transportiert wird, wobei
- die größte Dimension der Zellhohlräume (26) der zweidimensionalen Matrixstruktur kleiner ist als die durchschnittliche Breite des Kapillarkanals (13, 44),
- die Zellhohlräume (26) senkrecht zu der zweidimensionalen Ausdehnung der Matrixstruktur (24) eine definierte Höhe haben, durch die der Mindestwert der Schichtdicke der Flüssigkeitsschicht gewährleistet ist und
- die Zellen (25) an einer an die Flüssigkeitseintrittsseite des Testfeldes (5) angrenzende Übertragungsseite (28) der Matrixstruktur (24) offen sind.

7. Testelement nach Anspruch 6, **dadurch gekennzeichnet, dass** die zweidimensionale Matrixstruktur (24) parallel und benachbart zu einer Teillänge des Kapillarkanals (15, 43) angeordnet ist, und die Zellen (25) der Matrixstruktur (24) an einer dem Kapillarkanal (15, 43) zugewandten, der Übertragungsseite (28) gegenüberliegenden Flüssigkeitseintrittsseite (29) Zellöffnungen (33) aufweisen, so dass die Flüssigkeit durch diese Zellöffnungen (33) in die Zelfhahlräume (26) der Matrixstruktur (24) eindringt.

8. Testelement nach einem der Ansprüche 6 oder 7, **dadurch gekennzeichnet, dass** die Matrixstruktur (24) wenigstens 20, bevorzugt wenigstens 25, besonders bevorzugt wenigstens 50 und noch weiter bevorzugt wenigstens 100 Zellen (25) umfasst.

9. Testelement nach einem der vorhergehenden Ansprüche **dadurch gekennzeichnet, dass** die Dicke der Testschicht maximal 20 µm beträgt.

10. Testelement nach einem der Ansprüche 6 bis 9, **dadurch gekennzeichnet, dass** die Matrixstruktur (24) einstückig mit dem Stechelement (4) ausgebildet ist.

11. Testelement nach einem der Ansprüche 6 bis 10, **dadurch gekennzeichnet, dass** sich die Zellhohlräume (26) der Matrixstruktur (24) zur der Übertragungsseite (28) hin erweitern, vorzugsweise stetig erweitern.

12. Testelement nach einem der Ansprüche 6 bis 11, **dadurch gekennzeichnet, dass** der Kapillarkanal (15, 43) und die Matrixstruktur (24) in einem gemeinsamen hersteilungsverfahrensschritt durch Ätzen oder durch Ätzen und Laserschneiden hergestellt sind.

13. Analysesystem zur Analyse einer Körperflüssigkeit aus einem Körperteil umfassend ein Testelement nach einem der vorhergehenden Ansprüche und ein Analysegerät (2) mit
- einem Gehäuse (6),
- einem Kopplungsmechanismus (7) zur Ankopplung des Testelementes (4) an einen Antriebsmechanismus des Analysegeräts, um es auf einem Bewegungsweg einer Einstichbewegung zu bewegen, und
- einer Mess- und Auswerteeinheit (8) zur Messung einer Änderung eines mit der Körperflüssigkeit reagierenden Reagenz, um einen Analyten in der Körperflüssigkeit zu analysieren.

## Claims

1. Test element for generating a puncture wound in a body part, for receiving a body fluid sample from the body part, and for analizing by means of a reagent system, whose reaction with an analyte contained in the body fluid results in an optically measurable change of a measuring variable, which is characteristic for the desired analytical result, on the test element,
comprising a puncture element (4) and a test panel (5), which contains at least a part of the reagent system, wherein
the puncture element (4) has a tip positioned at one end of the puncture element (4) for generating a wound in the body part and a capillary structure (13, 44), which is implemented so that after the tip of the puncture element pierces into the skin, body fluid penetrates into the capillary structure (13, 44),
the puncture element (4) and the test panel (5) can be positioned relative to one another in a liquid transfer position so that the test panel is in fluid contact with a part of the capillary structure of the puncture element, body fluid which has penetrated into the capillary structure being able to be transferred to the test panel,
the test panel (5) comprises a transparent nonporous support layer (45, 100) and a test layer (101), which is applied to the support layer (45, 100) by coating and which does not penetrate into the support layer (45, 100), wherein the side of the test layer (101) facing away from the support layer (45, 100) forming the liquid entry side (104), which faces toward the capillary structure (13) in the liquid transfer position, and
in the liquid transfer position, the liquid entry side (104) of the test layer (101) directly adjoins the capillary structure (13, 44) in such a manner that a direct liquid transfer occurs from the capillary structure (13, 44) into the test layer (101).

2. Test element according to any one of the preceding claims, **characterized in that** the test panel is fixed on the puncture element, so that the puncture element and the test panel are permanently in fluid contact.

3. Test element according to any one of the preceding claims, **characterized in that** the test layer (101) includes two partial layers, a first partial layer coated on the support layer being a reaction layer (102) and a second partial layer coated thereon being an opaque light blocking layer (103).

4. Test element according to claim 3, **characterized in that** both the reaction layer (102) and also the opaque light blocking layer (103) contain an enzyme and a coloration reagent (indicator), the quantity of the enzyme in the reaction layer (102) being greater than that in the light blocking layer (103) and the quantity of the indicator in the reaction layer (102) being less than that in the light blocking layer (103).

5. Test element according to any one of the preceding claims, **characterized in that** at least one component of the reagent system, preferably an enzyme, is bound to another component of the test layer (191), in particular to a filler contained in the test layer (102).

6. Test element according to any one of the preceding claims, **characterized in that** the capillary structure (13, 44) includes a two-dimensional matrix structure (24) having a plurality of cells (25) having cell cavities (26) and a capillary channel, through which body fluid is transported to the two-dimensional matrix structure after the piercing of the tip of the puncture element (4) into the skin, wherein
- the largest dimension of the cell cavities (26) of the two-dimensional matrix structure being less than the average width of the capillary channel (13, 44),
- the cell cavities (26) having a defined height perpendicularly to the two-dimensional extension of the matrix structure (24), by which the minimum value of the layer thickness of the liquid layer is ensured, and
- the cells (25) being open on a transfer side (28) of the matrix structure (24), which adjoins the liquid entry side of the test panel (5).

7. Test element according to claim 6, **characterized in that** the two-dimensional matrix structure (24) is positioned parallel and adjacent to a partial length of the capillary channel (15, 43), and the cells (25) of the matrix structure (24) have cell openings (33) on a liquid entry side (29), which faces toward the capillary channel (15, 43) and is opposite to the transfer side (28), so that the liquid penetrates through these cell openings (33) into the cell cavities (26) of the matrix structure (24).

8. Test element according to any one of claims 6 or 7, **characterized in that** the matrix structure (24) comprises at least 20, preferably at least 25, particularly preferably at least 50, and still more preferably at least 100 cells (25).

9. Test element according to any one of the preceding claims, **characterized in that** the thickness of the test layer is at most 20 µm.

10. Test element according to any one of claims 6 to 9, **characterized in that** the matrix structure (24) is implemented integrally with the puncture element (4).

11. Test element according to any one of claims 6 to 10, **characterized in that** the cell cavities (26) of the matrix structure (24) widen toward the transfer side (28), preferably widen continuously.

12. Test element according to any one of claims 6 to 11, **characterized in that** the capillary channel (15, 43) and the matrix structure (24) are produced in a common production method step by etching or by etching and laser cutting.

13. Analysis system for analyzing a body fluid from a body part comprising a test element according to any one of the preceding claims and an analysis device (2) having
- a housing (6),
- a coupling mechanism (7) for coupling the lest element (4) to a drive mechanism of the analysis device, to move it on a movement path of a piercing movement, and
- a measuring and evaluation unit (8) for measuring a change of a reagent which reacts with the body fluid, in order to analyze an analyte in the body fluid.

## Revendications

1. Elément de test pour provoquer une plaie par piqûre dans une partie du corps, en vue du recueil d'un échantillon de liquide corporel provenant de la partie du corps et en vue d'une analyse au moyen d'un système de réactifs, dont la réaction avec un analyte contenu dans le liquide corporel conduit à une modification d'une grandeur de mesure caractéristique mesurable optiquement au niveau de l'élément de test pour le résultat analytique souhaité,
comprenant un élément de piqûre (4) et une zone de test (5), qui contient au moins une partie du système de réactifs, dans lequel l'élément de piqûre (4) présente un embout pointu disposé à une extrémité de l'élément de piqûre (4) pour provoquer une plaie dans la partie du corps et une structure capillaire (13, 44), qui est réalisée de sorte qu'après la piqûre de l'embout pointu de l'élément de piqûre dans la peau du liquide corporel pénètre dans la structure capillaire (13, 44),
l'élément de piqûre (4) et la zone de test (5) sont positionnables relativement l'un par rapport à l'autre dans une position de transfert de liquide corporel, de sorte que la zone de test est en contact fluidique avec une partie de la structure capillaire de l'élément de piqûre, le liquide corporel qui a pénétré dans la structure capillaire pouvant être transféré à la zone de test,
la zone de test (5) comprend une couche de support (45, 100) non poreuse transparente et une couche de test (101) appliquée par revêtement sur la couche de support (45, 100) et qui ne pénètre pas dans la couche de support (45, 100), le côté de la couche de test (101) opposé à la couche de support (45, 100) formant le côté d'entrée de liquide corporel (104) qui est tourné dans la position de transfert de liquide corporel de la structure capillaire (13) et dans la position de transfert de liquide corporel le côté d'entrée de liquide corporel (104) de la couche de test (101) est directement adjacent à la structure capillaire (13, 44), de telle sorte qu'un débordement de liquide corporel direct de la structure capillaire (13, 44) dans la couche de test (101) se produit.

2. Elément de test selon l'une des revendications précédentes, **caractérisé en ce que** la zone de test est fixée à l'élément de piqûre, de sorte que l'élément de piqûre et la zone de test sont en permanence en contact fluidique.

3. Elément de test selon l'une des revendications précédentes, **caractérisé en ce que** la couche de test (101) renferme deux couches partielles, une première couche partielle revêtue sur la couche de support étant une couche de réaction (102) et une deuxième couche partielle revêtue dessus étant une couche de blocage de la lumière opaque (103).

4. Elément de test selon la revendication 3, **caractérisé en ce qu'**aussi bien la couche de réaction (102) que la couche de blocage de la lumière opaque (103) contient une enzyme et un réactif chromogène (indicateur), la quantité de l'enzyme dans la couche de réaction (102) étant supérieure à celle de la couche de blocage de la lumière (103) et la quantité de l'indicateur dans la couche de réaction (102) étant inférieure à celle de la couche de blocage de la lumière (103).

5. Elément de test selon l'une des revendications précédentes, **caractérisé en ce qu'**au moins un composant du système de réactifs, de préférence une enzyme, est lié à un autre composant de la couche de test (191), en particulier une matière de remplissage contenue dans la couche de test (102).

6. Elément de test selon l'une des revendications précédentes, **caractérisé en ce que** la structure capillaire (13, 44) renferme une structure matricielle bidimensionnelle (24) comprenant une pluralité de cellules (25) avec des cavités de cellule (26) et un canal capillaire, à travers lequel après la piqûre de l'embout pointu de l'élément de piqûre (4) dans la peau du liquide corporel est transporté vers la structure matricielle bidimensionnelle, dans lequel
- la plus grande dimension des cavités de cellule (26) de la structure matricielle bidimensionnelle est inférieure à la largeur moyenne du canal capillaire (13, 44),
- les cavités de cellule (26) ont perpendiculairement à l'étendue bidimensionnelle de la structure matricielle (24) une hauteur définie qui permet de garantir la valeur minimale de l'épaisseur de couche de la couche de liquide et
- les cellules (25) sont ouvertes sur un côté de transfert (28) de la structure matricielle (24) adjacent au côté d'entrée de liquide corporel de la zone de test (5).

7. Elément de test selon la revendication 6, **caractérisé en ce que** la structure matricielle bidimensionnelle (24) est disposée parallèlement à et à côté d'une longueur partielle du canal capillaire (15, 43), et les cellules (25) de la structure matricielle (24) présentent des ouvertures de cellule (33) sur un côté d'entrée de liquide corporel (29) opposé au côté de transfert (28) et tourné vers le canal capillaire (15, 43), de sorte que le liquide pénètre dans les cavités de cellule (26) de la structure matricielle (24) par ces ouvertures de cellule (33).

8. Elément de test selon l'une des revendications 6 ou 7, **caractérisé en ce que** la structure matricielle (24) comprend au moins 20, de préférence au moins 25, plus préférablement au moins 50 et encore plus préférablement au moins 100 cellules (25).

9. Elément de test selon l'une des revendications précédentes, **caractérisé en ce que** l'épaisseur de la couche de test est au maximum de 20 µm.

10. Elément de test selon l'une des revendications 6 à 9, **caractérisé en ce que** la structure matricielle (24) est réalisée d'un seul tenant avec l'élément de piqûre (4).

11. Elément de test selon l'une des revendications 6 à 10, **caractérisé en ce que** les cavités de cellule (26) de la structure matricielle (24) s'élargissent dans la direction du côté de transfert (28), de préférence en continu.

12. Elément de test selon l'une des revendications 6 à 11, **caractérisé en ce que** le canal capillaire (15, 43) et la structure matricielle (24) sont fabriqués lors d'une étape de procédé de fabrication commune par gravure ou par gravure et découpe au laser.

13. Système d'analyse pour l'analyse d'un liquide corporel provenant d'une partie du corps comprenant un élément de test selon l'une des revendications précédentes et un appareil d'analyse (2) avec
- un boîtier (6),
- un mécanisme de couplage (7) pour le couplage de l'élément de test (4) à un mécanisme d'entraînement de l'appareil d'analyse pour le déplacer le long d'une trajectoire de déplacement d'un mouvement de piqûre, et
- une unité de mesure et d'évaluation (8) pour la mesure d'une modification d'un réactif réagissant avec le liquide corporel pour analyser un analyte dans le liquide corporel.
